(19) Europäisches Patentamt
European Patent Office
Office européen des brevets

(11) Veröffentlichungsnummer: **0 212 216**
**A1**

(12) **EUROPÄISCHE PATENTANMELDUNG**

(21) Anmeldenummer: **86109476.1**

(22) Anmeldetag: **11.07.86**

(51) Int. Cl.⁴: **C 07 D 215/22**, C 07 D 401/12, C 07 D 491/056, A 61 K 31/47

(30) Priorität: **20.07.85 DE 3526044**

(43) Veröffentlichungstag der Anmeldung: **04.03.87 Patentblatt 87/10**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Henning, Rainer, Dr., Rotenhofstrasse 31, D-6234 Hattersheim am Main (DE)**
Erfinder: **Lerch, Ulrich, Dr., Vorderwart 24, D-6238 Hofheim am Taunus (DE)**
Erfinder: **Kaiser, Joachim, Dr., Fichtestrasse 12, D-6000 Frankfurt am Main (DE)**

(54) **Dihydrochinolinon-Derivate, Verfahren zu ihrer Herstellung, sie enthaltende Arzneimittel und deren Verwendung, sowie Zwischenprodukte zu ihrer Herstellung.**

(57) Verbindungen der Formel I

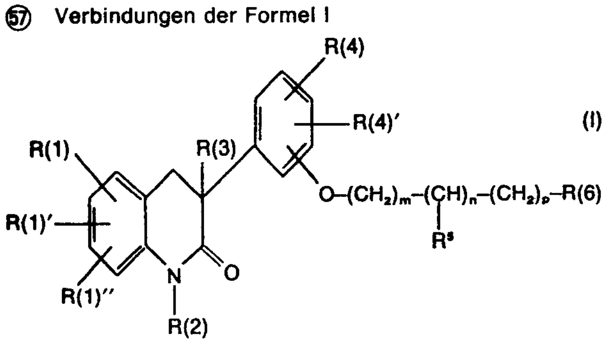

(I)

mit R(1), R(2), R(3), R(4) und R(5) unter anderem Wasserstoff und Alkyl, mit m 1–4 in 0 oder 1, p 0–4 und R(6) gleich verschiedenen Dialkylaminabkömmlingen, sowie deren Salze weisen calciumantagonistische Wirkung auf. Sie werden durch Umsetzung von entsprechenden Aminen mit einer Verbindung II erhalten, die an der Seitenkette eine nucleophil verdrängbare Abgangsgruppe trägt, oder aus einer Hydroxyphenyl-dihydrochinolin-2-on-Verbindung durch Umsetzung mit einer entsprechenden Seitenkettenverbindung, die eine nucleophil verdrängbare Abgangsgruppe aufweist.

EP 0 212 216 A1

# Dihydrochinolinon-Derivate, Verfahren zu ihrer Herstellung, sie enthaltende Arzneimittel und deren Verwendung, sowie Zwischenprodukte zu ihrer Herstellung

Es ist bekannt, daß Verbindungen, die das Einströmen von Calcium-Ionen in Zellen behindern, als Therapeutika zur Behandlung von verschiedenen Krankheiten, insbesondere des Herz-Kreislauf-Systems beim Menschen und anderen Warmblütern eingesetzt werden können.

In der britischen Patentschrift 1 305 278 sowie der deutschen Offenlegungsschrift 2 007 468 werden 3,4-Dihydro-3-phenyl-1-methyl-chinolin-2-one als Zentralnervensystem-Stimulantien beschrieben.

In der Niederländischen Patentanmeldung 6 516 320 wird 1-(2-Dimethylaminoethyl)-3-phenyl-tetrahydro-chinolin-2-on mit antihypertensiver Wirkung aufgeführt.

Bisher wurden jedoch keine Chinolin-2-one mit calciumantagonistischer Wirkung beschrieben, ebenfalls keine der erfindungsgemäßen Verbindungen.

Gegenstand der Erfindung sind Dihydrochinolinon-Derivate der Formel I, die eine calciumantagonistische Wirkung aufweisen,

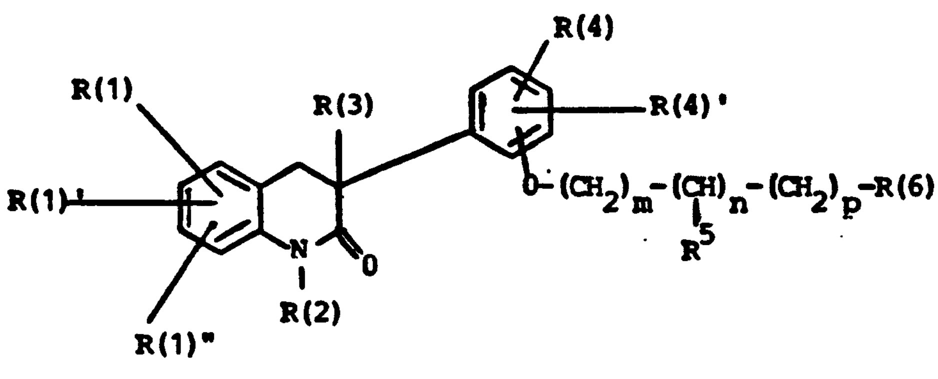

(I)

und in welcher

R(1), R(1)' und R(1)'' gleich oder verschieden und voneinander unabhängig sind und Wasserstoff, $(C_1-C_4)$-Alkyl, $(C_1-C_3)$-Alkoxy, F, Cl, $CF_3$, Nitro, Hydroxy, Acetamido oder Amino, oder R(1) und R(1)' gemeinsam $(C_1-C_2)$-alkylendioxy,

R(2) Wasserstoff, $(C_1-C_{10})$-Alkyl, geradkettig oder verzweigt, $(C_3-C_{10})$-Alkenyl, geradkettig oder verzweigt,

R(3) Wasserstoff, $(C_1-C_{10})$-Alkyl, geradkettig oder verzweigt, $(C_3-C_{10})$-Alkenyl, geradkettig oder verzweigt, $(C_4-C_8)$-Cycloalkyl, $(C_4-C_8)$-Cycloalkyl-$(C_1-C_4)$-alkyl, Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)' gleich oder verschieden und voneinander unabhängig Wasserstoff, $(C_1-C_4)$-Alkyl, $(C_1-C_3)$-Alkoxy, F, Cl, $CF_3$, Nitro, Hydroxy, Acetamido oder Amino,

R(5) Wasserstoff, Hydroxy oder $(C_1-C_3)$-Alkyl,

m 1, 2, 3 oder 4,

n 0 oder 1,

p 0, 1, 2, 3 oder 4

bedeuten,

und

R(6) eine der folgenden Gruppen bedeutet,

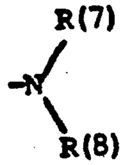 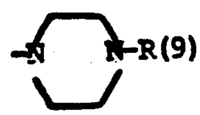 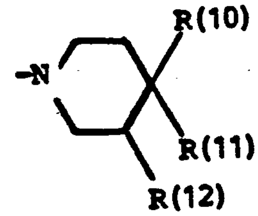 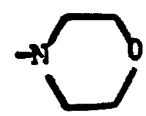

worin

R(7) und R(8) gleich oder verschieden und voneinander unabhängig Wasserstoff, $(C_1-C_{10})$-Alkyl, $(C_4-C_8)$-Cycloalkyl, $(C_4-C_8)$-Cycloalkyl-$(C_1-C_4)$-alkyl, Phenyl-$(C_1-C_6)$-alkyl, wobei der Phenylrest unsubstituiert oder durch einen, zwei oder drei Reste aus der Gruppe $(C_1-C_4)$-Alkylendioxy, F, Cl, $CF_3$ oder Hydroxy substituiert ist, Pyridyl-$(C_1-C_4)$-alkyl

R(9) Wasserstoff, $(C_1-C_{10})$-Alkyl, geradkettig oder verzweigt, Phenyl, Phenyl-$(C_1-C_4)$-alkyl oder Diphenyl-$(C_1-C_5)$-alkyl, Phenyl-$(C_1-C_4)$-alkanoyl, Benzoyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl, Pyrimidinyl, $(C_1-C_5)$-Alkanoyl,

R(10) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(11) Wasserstoff, Hydroxy, $(C_1-C_4)$-Alkoxy oder zusammen mit R(12) eine Bindung, und

R(12) Wasserstoff oder zusammen mit R(11) eine Bindung bedeuten;

sowie die Salze der Verbindungen der Formel I mit pharmazeutisch akzeptablen Säuren.

Bevorzugt werden Verbindungen der Formel I, in welcher

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido; oder gemeinsam $(C_1-C_2)$alkylendioxy,

R(1)" Wasserstoff

R(2) Wasserstoff, $(C_1-C_6)$-Alkyl, geradkettig oder verzweigt,

R(3) Wasserstoff, $(C_1-C_6)$-Alkyl, geradkettig oder verzweigt, Allyl, Cyclopentyl, Cyclohexyl, Benzyl, Phenylethyl, jeweils unsubstituiert oder durch $(C_1-C_3)$-Alkyl, $(C_1-C_3)$-Alkoxy, F, Cl, $CF_3$, $(C_1-C_2)$-Alkylendioxy oder Nitro substituiert,

R(4) Wasserstoff, Methyl, Methoxy, Ethoxy, Chlor, Nitro, Hydroxy, Acetamido oder Amino,

R(4)' Wasserstoff,

R(5) Wasserstoff oder Hydroxy,

m 1, 2 oder 3,

n 0 oder 1,

p 1, 2, 3 oder 4 bedeuten und

R(6) eine der folgenden Gruppen

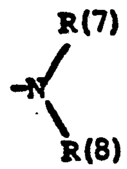
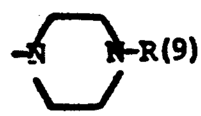
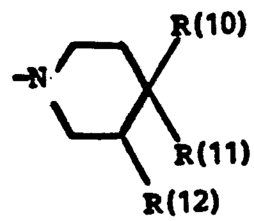

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-$(C_1-C_4)$-alkyl, wobei der Phenylrest unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl-$(C_1-C_4)$-alkyl,

R(9) wie oben angegeben definiert ist,

R(10) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(11) Wasserstoff und Hydroxy, Methoxy oder zusammen mit R(12) eine Bindung,

R(12) Wasserstoff oder zusammen mit R(11) eine Bindung bedeuten;

sowie die Salze dieser Verbindungen der Formel I mit pharmazeutisch akzeptablen Säuren.

Besonders bevorzugt werden Verbindungen der Formel I, in welcher

R(1) Wasserstoff, Methyl, Methoxy, Fluor oder Chlor,
R(1)' und R(1)'' Wasserstoff,
R(2) Wasserstoff, Methyl, Ethyl,
R(3) Wasserstoff, Methyl, Ethyl, Propyl, Isopropyl, Butyl, sec. Butyl, Isobutyl, Allyl, Cyclopentyl, Cyclohexyl, Benzyl, Phenylethyl, wobei der Phenylring jeweils unsubstituiert oder durch Methyl, Methoxy, Chlor, Fluor, Methylendioxy, Nitro substituiert ist,
R(4) Wasserstoff, Methyl, Methoxy, Chlor, Nitro oder Hydroxy,
R(4)' Wasserstoff,
R(5) wie oben definiert
m 0 oder 1,
n 0 oder 1,
p 1, 2, 3 oder 4
bedeuten, und
R(6) eine der folgenden Gruppen bedeutet

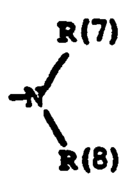

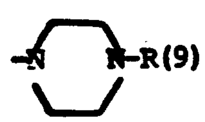

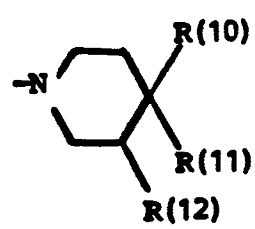

worin

R(7) Wasserstoff oder Methyl,

R(8) Phenyl-($C_1$-$C_4$)-alkyl, wobei der Phenylrest unsubstituiert oder durch einen, zwei oder drei Reste aus der Gruppe Methyl, Methoxy, Chlor, Methylendioxy oder Hydroxy substituiert ist,

R(9) wie oben definiert ist,

R(10) Phenyl oder Phenyl-($C_1$-$C_2$)-alkyl, wobei der Phenylrest jeweils unsubstituiert oder durch einen, zwei oder drei Reste aus der Gruppe Methyl, Methoxy, Chlor, Methylendioxy oder Hydroxy substituiert ist,

R(11) Wasserstoff, Hydroxy, Methoxy oder zusammen mit R(12) eine Bindung, und

R(12) Wasserstoff oder zusammen mit R(11) eine Bindung bedeuten;

sowie die Salze dieser Verbindungen der Formel I mit pharmazeutisch akzeptablen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Insbesondere bevorzugt sind Verbindungen der Formel I wie vorstehend definiert, in denen

R(1) Wasserstoff, Methyl, Methoxy, Fluor oder Chlor,

R(1)' und R(1)" Wasserstoff,

R(2) Wasserstoff, Methyl,

R(3) Methyl, Ethyl, Propyl, Isopropyl, Butyl, sec. Butyl, Isobutyl, Cyclopentyl, Cyclohexyl, Benzyl, unsubstituiert oder durch Methoxy, Methyl, Fluor, Chlor, oder Nitro substituiert,

R(4) Wasserstoff, Methoxy, Methyl oder Chlor,
R(5) Wasserstoff oder Hydroxy,
m 0 oder 1
n 0 oder 1
p 1, 2, 3 oder 4
R(6) eine der folgenden Gruppen

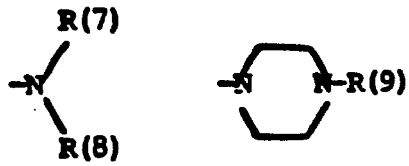

R(7) Methyl,
R(8) Phenyl-($C_1$-$C_4$)-alkyl, wobei der Phenylrest unsubstituiert oder durch einen, zwei oder drei Reste aus der Gruppe Methyl, Methoxy, Chlor, Methylendioxy oder Hydroxy substituiert ist,
R(9) Phenyl-($C_1$-$C_4$)-alkyl, wobei der Phenylrest unsubstituiert oder durch einen, zwei oder drei Reste aus der Gruppe ($C_1$-$C_2$)-Alkoxy, ($C_1$-$C_2$)-Alkylendioxy oder Hydroxy substituiert ist, Diphenyl-($C_1$-$C_4$)-alkyl, wobei die Phenylreste unsubstituiert oder durch Chlor, Fluor oder Methoxy substituiert sind,

und deren physiologisch verträgliche Salze.

Halogen bedeutet, wenn nicht anders angegeben Fluor oder Chlor.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Gemische von Diastereomeren

können nach gebräuchlichen Methoden, zum Beispiel durch selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin ein Verfahren zur Herstellung von Verbindungen der Formel I, das dadurch gekennzeichnet ist, daß man

a) eine Verbindung der Formel II,

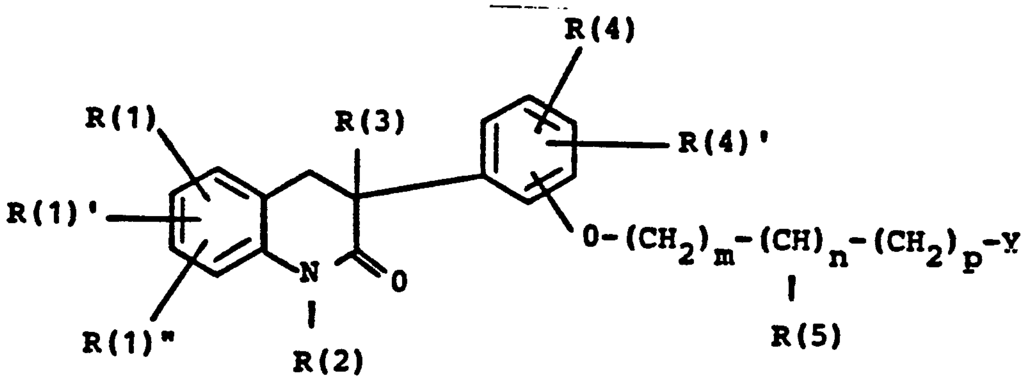
(II)

in welcher R(1), R(1)', R(1)", R(2), R(3), R(4), R(4)', R(5), m, n und p die gleiche Bedeutung wie in Formel I haben, und in welcher Y eine Abgangsgruppe, die nucleophil verdrängt werden kann, insbesondere ein Cl-, Br- oder J-Atom, einen Sulfonsäurerest, vorzugsweise einen Methansulfonylrest, einen Benzolsulfonylrest, einen Toluolsulfonylrest oder einen Trifluormethansulfonylrest bedeutet,
mit einer der Verbindungen der Formeln IIIa, IIIb, IIIc oder IIId,

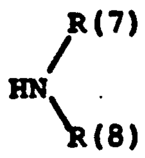 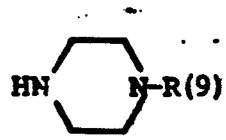 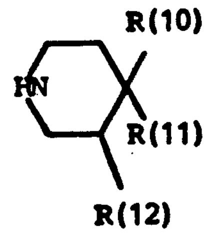 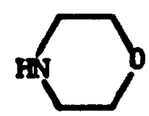

(IIIa) (IIIb) (IIIc) (IIId)

in welchen R(7), R(8), R(9), R(10), R(11) und R(12) die gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160°C, vorzugsweise zwischen 20 und 120°C, umsetzt, oder daß man

b) eine Verbindung der Formel IV,

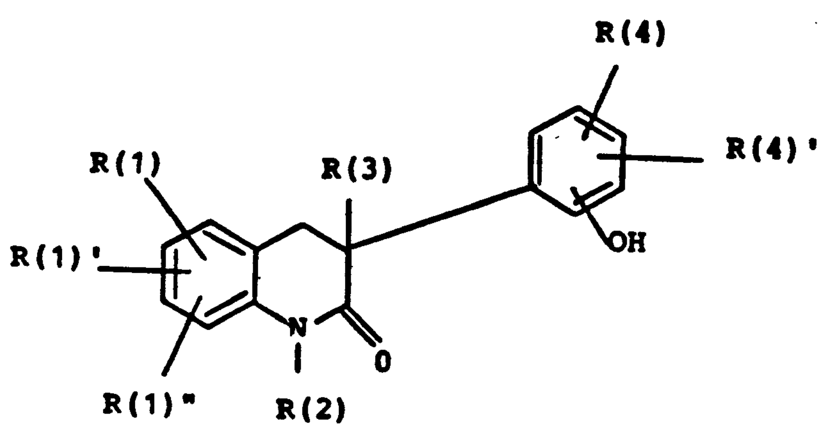

(IV)

in welcher R(1), R(1)', R(1)", R(2), R(3), R(4), R(4), R(4)' die gleiche Bedeutung wie in Formel I haben, mit einer Verbindung der Formel V,

$$Z-(CH_2)_m-(\underset{R(5)}{\underset{|}{CH}})_n-(CH_2)_p-R(6) \qquad (V)$$

in welcher Z gleich wie Y in Formel II definiert ist und in welcher R(5), R(6), m, n und p die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen -40 und +60°C, vorzugsweise zwischen -10 und +30°C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispielsweise Triethylamin, N-Ethylmorpholin, N-Methyldiisopropylamin oder Pyridin, bei einer Temperatur zwischen 0 und 160°C, vorzugsweise zwischen 20 und 120°C, umsetzt.

Verbindungen der Formel II erhält man durch Umsetzung von 2-Nitrobenzaldehyden der Formel VI, in welcher R(1), R(1)' und R(1)" die gleiche Bedeutung wie in Formel I haben

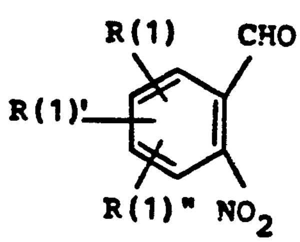

(VI)

mit Phenylessigsäuren der Formel VII

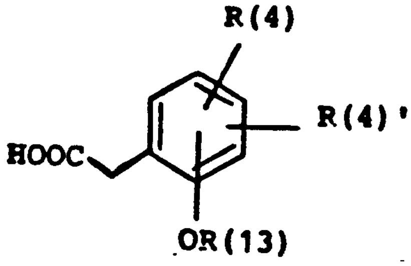

(VII)

in welcher R(4) und R(4)' die gleiche Bedeutung wie in Formel I haben und R(13) einen unter milden Bedingungen abspaltbaren Rest wie beispielsweise einen Methyl-, Benzyl- oder Acetylrest darstellt, in Essigsäureanhydrid bei 60 bis 130°C mit oder ohne Zusatz eines tertiären Amins wie beispielsweise Tripropylamin, Diisopropylethylamin oder N-Methylmorpholin, wobei Verbindungen der Formel VIII erhalten werden

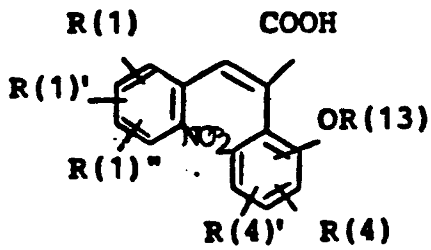

VIII

Hydrierung mit einem Edelmetallkatalysator wie beispielsweise Palladium auf Tierkohle oder mit Raney-Nickel in einem alkoholischen Lösungsmittel oder einem Ester wie beispielsweise Ethanol oder Essigsäureethylester unter 1 bis 30 bar $H_2$-Druck bei 0 bis 60°C ergibt Verbindungen der Formel IX

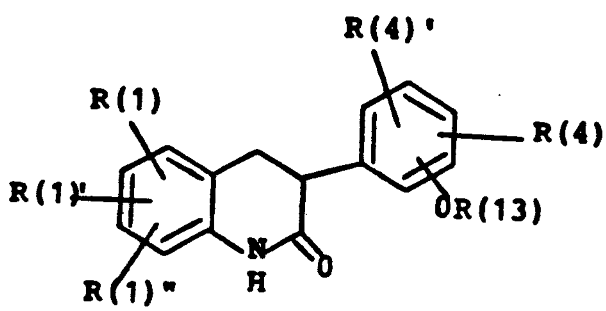

IX

Alkylierung am Stickstoff erfolgt durch Umsetzung mit einer Base wie Kaliumcarbonat oder einem Alkalihydrid und einem Alkylhalogenid der Formel R(2)-Hal, worin R(2) wie zu Formel I definiert ist und Hal Cl, Br oder Jod bedeutet, zu Verbindungen der Formel X

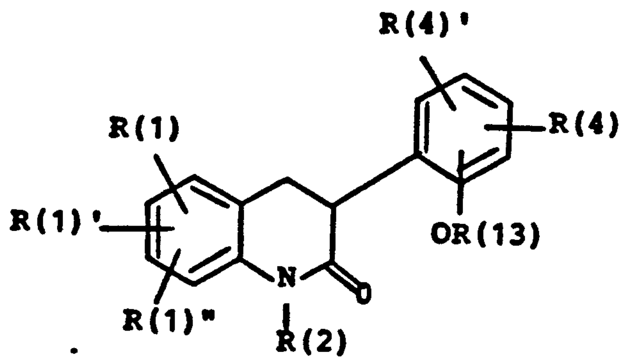

X

In Verbindungen der Formel X können Reste R(3) durch Alkylierung mit einem Alkylhalogenid der Formel R(3)-Hal, in welcher R(3) wie zu Formel I definiert ist und Hal Cl, Br oder Jod bedeutet, in Gegenwart einer starken Base wie Natriumhydrid, Kaliumhydrid oder einem Alkaliamid eingeführt werden, wobei man Verbindungen der Formel XI erhält:

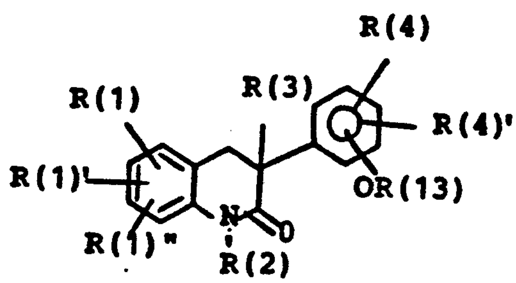

XI

Anschließend wird die Schutzgruppe R(13) unter geeigneten Bedingungen, so beispielsweise durch katalytische Hydrierung für die Benzylgruppe, Umsetzung mit Bortribromid, Trimethyljodsilan oder Pyridinhydrochlorid für die Methylgruppe oder Kaliumcarbonat in alkoholischer Lösung für die Acetylgruppe, abgespalten. Es entstehen Verbindungen der Formel IV.

Die Verbindungen der Formel IV können anschließend unter den für Verfahrensvariante b) beschriebenen Bedingungen mit einer Verbindung der Formel XII

$$Z-(CH_2)_m-\underset{\displaystyle R(5)}{(CH)_n}-(CH_2)_p-Y \qquad (XII)$$

in welcher R(5), m, n und p die gleiche Bedeutung wie in Formel I haben, Y die gleiche Bedeutung wie in Formel II hat und Z wie Y definiert ist, wobei Z und Y gleich oder verschieden sind,
zu den Verbindungen der Formel II umgesetzt werden.

Verbindungen der Formel V erhält man in an sich bekannter Weise aus Verbindungen der Formeln IIIa, IIIb, IIIc oder IIId durch Umsetzung mit Verbindungen der Formel XII unter den bei Verfahrensvariante a) beschriebenen Bedingungen.

Die erfindungsgemäßen Verbindungen der Formel I weisen pharmakologische und biochemische Wirkungen, insbesondere calciumantagonistische Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkanäle enthalten, mit der markierten Substanz beladen. Nach Inkubation mit der Testsubstanz wird die freigesetzte Radioaktivität in der überstehenden Lösung bestimmt. In diesem Modell weisen die erfindungsgemäßen Verbindungen der Formel I $IC_{50}$-Werte von $10^{-6}$ molar bis $10^{-10}$ molar auf.

In weiteren Testmodellen, mit denen calciumantagonistische Wirkung nachgewiesen werden kann, z.B. an der Coronardurchströmung am isolierten Meerschweinchenherzen oder am Aktionspotential des isolierten Meerschweinchenpapillarmuskels sind die Verbindungen der Formel I ebenfalls stark wirksam.

Die erfindungsgemäßen Verbindungen der Formel I und ihre pharmakologisch verträglichen Salze vermindern das Einströmen von Calcium-Ionen in Zellen und eignen sich daher zur Behandlung des Herz-Kreislaufsystems bei entsprechenden Beschwerden, z.B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise vom Zustand, vom Typ und von der Größe des behandelten Säugers. Bei oraler Dosierung werden befriedigende Ergebnisse mit Dosen von ab 0,01, vorzugsweise ab 0,1, insbesondere ab 0,5 mg und bis zu 100 mg, vorzugsweise bis zu 20 mg, insbesondere bis zu 15 mg einer Verbindung der Formel I pro kg Körpergewicht erreicht. Beim Menschen variiert die tägliche Dosis von mindestens 10, insbesondere 20 mg, bis höchstens 800 mg, vorzugsweise 500 mg wobei Einzeldosen von 5 bis 200 mg, insbesondere 5 - 100 mg vorzugsweise ein bis dreimal täglich, gegeben werden können.

Für intravenöse und intramuskuläre Anwendung beträgt die Dosis mindestens 1 mg, vorzugsweise 5 und höchstens 300 mg, vorzugsweise 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine Traganth, Methylcellulose, Natriumcarboxymethylcellulose und/oder Polyvinylpyrrolidon und, falls benötigt, Farbstoff, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-, Granulier- und Dragierverfahren, hergestellt und enthalten 0,1 % bis etwa 75 %, bevorzugt etwa 1 % bis etwa 50 % des Wirkstoffs.

Die im Anschluß folgenden Beispiele sollen die Erfindung erläutern, ohne sie auf diese Beispiele zu begrenzen.

Beispiel 1

1H-3,4-Dihydro-3-(2-methoxyphenyl)-chinolin-2-on

a) 2-(2-Methoxyphenyl)-3-(2-nitrophenyl)-2-propensäure

25 g 2-Nitrobenzaldehyd und 28 g 2-Methoxyphenylessigsäure sowie 0.16 Mol Diisopropylethylamin werden in 90 ml Acetanhydrid 8 Stunden am Rückfluß unter Stickstoff gekocht. Nach Abkühlen auf 60 °C wird auf 200 ml heißes Wasser gegossen. Unter Kühlung wird noch 2 Stunden gerührt, das Produkt abgesaugt und mit wenig kaltem Ethanol gewaschen. Nach Umkristallisieren aus Ethanol erhält man 38.7 g der Titelverbindung vom Schmp. 217-219°C

b) 1H-3,4-Dihydro-3-(2-methoxyphenyl)-chinolin-2-on

38 g (0.127 Mol) 2-(2-Methoxyphenyl)-3-(2-nitrophenyl)-2-propensäure werden in 1 L Methanol gelöst und unter Zusatz von 3 g Palladium auf Tierkohle (5 %) unter 1 bar $H_2$-Druck hydriert (5 Stunden). Nach Absaugen des Katalysators wird eingeengt und aus Isopropylether kristallisiert. Man erhält 24.2 g der Titelverbindung als farblose Kristalle vom Schmp. 188-190°C.

$^1$H-NMR ($CDCl_3$) : $\delta$ = 8,5 (br. s, 1H); 7,3 - 6,6 (m,8H); 4,4 - 4,0 (X-Teil eines ABX-Systems,1H); 3,8 (s,3H); 3,6-2,9 (AB-Teil eines ABX-Systems, 2H) ppm.

Nach den in Beispiel 1 angegebenen Vorschriften wurden unter Verwendung der entsprechenden Ausgangsmaterialien hergestellt:

Beispiel 2

1H-3,4-Dihydro-3(3-methoxyphenyl)-chinolin-2-on

Farblose Kristalle von Schmp. 138°C

Beispiel 3

1H-3,4-Dihydro-3-(4-methoxyphenyl)-chinolin-2-on

Farblose Kristalle vom Schmp. 190°C

Beispiel 4

1H-3,4-Dihydroxy-1-methyl-3-(2-methoxyphenyl)-chinolin-2-on

3.09 g Natriumhydrid (50 % in Öl) werden mit n-Hexan gewaschen und dann in 100 ml trockenem Dimethylformamid suspendiert. Unter Eiskühlung gibt man 13.6 g (53.7mmol) 1H-3,4-Dihydro-3-(2-methoxyphenyl)-chinolin-2-on hinzu und rührt 15 Minuten. Nach Zugabe von 9.14 g Jodmethan wird 1 Stunde gerührt, auf Eiswasser gegossen, mit Essigester extrahiert, der Extrakt fünfmal mit Wasser gewaschen, mit Natriumsulfat getrocknet und eingeengt.Nach Verreiben mit Diisopropylether erhält man 12.6 g farblose Kristalle vom Schmp. 111-115°C.

Nach der in Beispiel 4 angegebenen Vorschrift werden ebenfalls hergestellt:

Beispiel 5

1H-3,4-Dihydro-1-methyl-3-(3-methoxyphenyl)-chinolin-2-on

Farblose Kristalle vom Schmp. 84-86°C

Beispiel 6

1H-3,4-Dihydro-1-methyl-3-(4-methoxyphenyl)-chinolin-2-on

Farblose Kristalle vom Schmp. 98-99°C

Beispiel 7

1H-3,4-Dihydro-1,3-dimethyl-3(2-methoxyphenyl)-chinolin-2-on

3,6 g Kaliumhydrid (50 % in Öl) werden mit n-Hexan gewaschen und in 40 ml trockenem Dimethylformamid suspendiert. Bei 0°C gibt man unter Stickstoff 3 g (11.2mmol) 1H-3,4-Dihydro-1-methyl-3-(2-methoxyphenyl)-chinolin-2-on zu. Nach 15 Minuten bei 0°C erfolgt die Zugabe von 3.18 g Jodmethan. Nach weiteren 2,5 Stunden bei Raumtemp. wird auf Eiswasser gegossen, mit Essigester extrahiert, mit Wasser gewaschen, mit Natriumsulfat getrocknet und eingeengt. Man erhält 3,3 g der Titelverbindung.

$^1$H-NMR ($CDCl_3$): $\delta$ = 7.3 - 6.6 (m,8H); 3.85 + 2.55 (AB-System,2H); 3.69 (s,3H); 3,43 (s,3H); 1,53 (s,3H) ppm.

Nach der in Beispiel 7 angegebenen Vorschrift wurden unter Verwendung der geeigneten Ausgangsmaterialien ebenfalls hergestellt:

Beispiel 8

1H-3,4-Dihydro-3-ethyl-1-methyl-3-(2-methoxyphenyl)-chinolin-2-on

Farblose Kristalle vom Schmp. 135 - 137° C

Beispiel 9

1H-3,4-Dihydro-3-isopropyl-1-methyl-3-(2-methoxyphenyl)-chinolin-2-on

Farbloses Öl.

$^1$H-NMR ($CDCl_3$): $\delta$ = 7.1 - 6.5 (m,8H); 3.6 + 3.05 (AB-System, 2H); 3.72 (s,3H), 3.39 (s,3H); 2.65 (septett, 1H); 1.05 und 1.0 (2d,6H) ppm.

Beispiel 10

1H-3,4-Dihydro-3-n-hexyl-1-methyl-3-(2-methoxyphenyl)-chinolin-2-on

Farbloses Öl

Beispiel 11

1H-3,4-Dihydro-3-benzyl-1-methyl-3-(2-methoxyphenyl)-chinolin-2-on

Farbloses Öl.

$^{1}$H-NMR ($CDCL_3$) : $\delta$ = 7.2- 6.5 (m,13H); 3.73 (s,3H); 3.36 (s,3H); 3.77 + 2.74 (AB-System, 2H); 3.56 (AB-System,2H) ppm.

Beispiel 12

1H-3,4-Dihydro-3-isopropyl-1-methyl-3-(3-methoxyphenyl)-chinolin-2-on

Farbloses Öl.

Beispiel 13

1H-3,4-Dihydro-3-benzyl-1-methyl-3-(3-methoxyphenyl)-chinolin-2-on

Farbloses Öl.

Beispiel 14

1H-3,4-Dihydro-3-isopropyl-1-methyl-3-(4-methoxyphenyl)-chinolin-2-on

Farblose Kristalle

Beispiel 15

1H-3,4-Dihydro-3-benzyl-1-methyl-3-(4-methoxyphenyl)-chinolin-2-on

Farblose Kristalle

Beispiel 16

1H-3,4-Dihydro-1,3-dimethyl-3-(2-hydroxyphenyl)-chinolin-2-on

3.3 g (11.7 mmol) 1H-3,4-Dihydro-1,3-dimethyl-3-(2-methoxyphenyl)-chinolin-2-on werden unter Eiskühlung in 40 ml trockenem Dichlormethan gelöst und mit 14 ml einer 1M Lösung von Bortribromid in n-Hexan tropfenweise versetzt. Nach 3 Stunden bei Raumtemperatur wird auf Eiswasser gegossen, nochmals mit Dichlormethan extrahiert und mit Natriumsulfat getrocknet. Kristallisation aus Isopropanol/Diisopropylether liefert 2.18 g der Titelverbindung als farblose Kristalle vom Schmp. 162 - 164° C

Analog der in Beispiel 16 angegebenen Vorschrift wurden unter Verwendung der entsprechenden Ausgangsverbindungen hergestellt:

Beispiel 17

1H-3,4-Dihydro-3-isopropyl-1-methyl-3(2-hydroxyphenyl)-chinolin-2-on

Farblose Kristalle vom Schmp. 206-208° C

Beispiel 18

1H-3,4-Dihydro-3-ethyl-1-methyl-3-(2-hydroxyphenyl)-chinolin-2-on

Farblose Kristalle vom Schmp. 171-173° C

Beispiel 19

1H-3,4-Dihydro-3-n-hexyl-1-methyl-3-(2-hydroxyphenyl)-chinolin-2-on

Farblose Kristalle vom Schmp. 110 - 111° C

Beispiel 20

1H-3,4-Dihydro-3-benzyl-1-methyl-3-(2-hydroxyphenyl)-chinolin-2-on

Farblose Kristalle

$^1$H-NMR ($CDCl_3$): $\delta$= 9.35 (s,1H); 7.3 - 6.5 (m,13H); 3.75 + 3.1 (AB-System, 2H); 3.40(s,3H); 3.1(s,2H) ppm.

Beispiel 21

1H-3,4-Dihydro-3-isopropyl-1-methyl-3-(3-hydroxyphenyl)-chinolin-2-on

Farblose Kristalle

Beispiel 22

1H-3,4-Dihydro-3-benzyl-1-methyl-3-(3-hydroxyphenyl)-chinolin-2-on

Farblose Kristalle

Beispiel 23

1H-3,4-Dihydro-3-isopropyl-1-methyl-3-(4-hydroxyphenyl)-chinolin-2-on

Farblose Kristalle

Beispiel 24

1H-3,4-Dihydro-3-benzyl-1-methyl-3-(4-hydroxyphenyl)-chinolin-2-on

Farblose Kristalle

Beispiel 25

1H-3.4-Dihydro-1.3-dimethyl-3-[2-(4-brombutoxy)-phenyl]-chinolin-2-on

2.2 g (8.2 mmol) 1H-3.4-Dihydro-1,3-dimethyl-3-(2-hydroxyphenyl)-chinolin-2-on, 1.57 g Kaliumcarbonat und 5.3 g 1,4-Dibrombutan werden in 30 ml Butanon-2 6 Stunden am Rückfluß gekocht. Nach Abkühlen werden die Salze abfiltriert und das Filtrat eingeent. Beim Verreiben mit Diisopropylether werden 2.99 g Kristalle vom Schmp. 246-249°C erhalten.

Analog der in Beispiel 25 angegebenen Vorschrift werden die nachfolgenden Verbindungen unter Verwendung der entsprechenden Ausgangsmaterialien hergestellt:

Beispiel 26

1H-3,4-Dihydro-3-ethyl-1-methyl-3-[2-(4-brombutoxy)-phenyl]-chinolin-2-on

Farbloses Öl.

$^1$H-NMR ($CDCl_3$): $\delta$ = 7.3 - 6.6 (m,8H); 4.2-3.8 (m,2H); 3,8 + 2.7 (AB-System, 2H); 3.55-3.2 (m+s,5H); 2.3-1.6 (m,6H); 0.92 (t,3H) ppm.

Beispiel 27

1H-3,4-Dihydro-3-n-hexyl-1-methyl-3-[2-(4-brombutoxy)-phenyl]-chinolin-2-on

Farbloses Öl.

$^1$H-NMR ($CDCl_3$): $\delta$ = 7.2 - 6.6 (m,8H); 4.0 (t,2H); 3.82 + 2.73 (AB-System, 2H); 3.6 - 3.3 (s+m, 5H); 2.3-1.7 (m,6H); 1.5-1.05 (m,8H); 0.85 (t,3H) ppm.

Beispiel 28

1H-3.4-Dihydro-3-benzyl-1-methyl-3-[2-(4-brombutoxy)-phenyl]-chinolin-2-on

Farblose Kristalle vom Schmp. 122-125° C

Beispiel 29

1H-3,4-Dihydro-3-isopropyl-1-methyl-3-[2-(4-brombutoxy)-phenyl]-chinolin-2-on

Farbloses Öl.

$^1$H-NMR ($CDCl_3$).$\delta$ = 7.1-6.5(m,8H); 3.9 (m,2H); 3.95 + 3.07 (AB-System, 2H); 2.65 (septett,1H); 2.3-1.8 (m,4H); 1.05 (d,6H)ppm

Beispiel 30

1H-3,4-Dihydro-3-isopropyl-1-methyl-3-[2-(3-brompropoxy)-phenyl]-chinolin-2-on

Farbloses Öl

Beispiel 31

1H-3.4-Dihydro-3-isopropyl-1-methyl-3-[2-(5-brompentoxy)-phenyl]-chinolin-2-on

Farbloses Öl

Beispiel 32

1H-3,4-Dihydro-3-isopropyl-1-methyl-3-[3-(3-brompropoxy)-phenyl]-chinolin-2-on

Farbloses Öl

Beispiel 33

1H-3,4-Dihydro-3-isopropyl-1-methyl-3-[4-(3-brompropoxy)-phenyl]-chinolin-2-on

Farbloses Öl

Beispiel 34

1H-3,4-Dihydro-3-isopropyl-1-methyl-3-[3-(4-brombutoxy)-phenyl]-chinolin-2-on

Farbloses Öl

Beispiel 35

1H-3,4-Dihydro-1-methyl-3-(2-hydroxyphenyl)-chinolin-2-on

Hergestellt nach der in Beispiel 16 angegebenen Vorschrift aus 1H-3,4-Dihydro-1-methyl-3-(2-methoxyphenyl)-chinolin-2-on und Bortribromid.
Farblose Kristalle vom Schmp. 160 - 161° C

Beispiel 36

1H-3.4-Dihydro-1-methyl-3-[2-(4-brombutoxyphenyl)]-chinolin-2-on

Hergestellt nach dem in Beispiel 25 beschriebenen Verfahren aus 1H-3,4-Dihydro-1-methyl-3-(2-hydroxyphenyl)-chinolin-2-on.
Farbloses Öl.

Beispiel 37

1H-3,4-Dihydro-1-methyl-3-[2-[4-[4-[2-(3,4,5-trimethoxyphenyl)-ethyl]-piperazinyl]-butoxy]-phenyl]-chinolin-2-on.Dihydrochlorid

2.7 g (5.6 mmol) 1H-3,4-Dihydro-1-methyl-3-[2-(4-brom-butoxy)-phenyl]-chinolin-2-on, 3.5 g (11.2 mmol) 2-(3,4,5-Trimethoxyphenyl)-ethyl-piperazin und 3.5 g Kaliumcarbonat werden in 50 ml Isopropanol 6 Stünden am Rückfluß gekocht. Nach Filtration wird eingeengt und an Kieselgel mit Dichlormethan/Methanol (20:1) chromatographiert. Man erhält 2.2 g farbloses Öl.

$^1$H-NMR ($CDCl_3$): $\delta$ = 7.3-6.7 (m,8H); 6.43 (s,2H); 4.3-3.5 (m,3H); 3.72 + 3.70 (2s,9H); 3.43 (s,3H); 3.5-3.0 (m,2H); 2.9-2.2 (m,14H); 2.0-1.6 (m,4H) ppm.

Die freie Base wird in Aceton aufgenommen und mit einem Überschuß 2.5 N ethanolischer HCL versetzt. Nach Einengen wird in Aceton aufgenommen und abgesaugt. 2.5 g farblose Kristalle vom Schmp. 228° C.

Analog dem in Beispiel 37 beschriebenen Verfahren werden die folgenden Verbindungen bei Verwendung der entsprechenden Ausgangsmaterialien erhalten:

## Beispiel 38

**1H-3,4-Dihydro-1-methyl-3-isopropyl-3-[2-[4-[4-[2-(3,4,5-trimethoxyphenyl)-ethyl] -piperazinyl] -butoxy] - phenyl-] chinolin-2-on.Dihydrochlorid**

Farblose Kristalle vom Schmp. 236° C

$^1$H-NMR (freie Base, $CDCl_3$): $\delta$ = 7.2-6.5 (m,8H) 6.41 (s,2H); 4,1-3.9 (m,2H); 3.83 + 3.81 (2s,9H); 3.68 + 3.03 (AB-System, J=15Hz, 2H); 3.37(s, 3H); 2.9 - 2.4 (m,15H); 2.0-1.5 (m,4H); 1.0 (d,6H) ppm.

$C_{38}H_{53}Cl_2N_3O_5$ (702.77) Ber. C 64.9 H 7.5 N 6.0
Gef. C 64.6 H 7.6 N 5.7

Beispiel 39

1H-3,4-Dihydro-1-methyl-3-benzyl-3-[2-[4-[4-[2-(3,4,5-trimethoxyphenyl)-ethyl] - piperazinyl] -butoxy] -phenyl] -chinolin-2-on. Dihydrochlorid

Farblose Kristalle vom Schmp. 210° C (Zersetzung)

$^1$H-NMR (freie Base, $CDCl_3$): δ = 7.2-6.5 (m,13H); 6.40 (s, 2H); 3,71 + 3.69 (2s,9H); 4.2- 3.6 (m,3H); 3.80 + 3.20 (AB-System,J= 14Hz,2H); 3,4 (s,3H), 3.0 - 2.2 (m,15H); 2.0 - 1.6 (m,4H) ppm.

$C_{42}H_{51}N_3O_5 \cdot 2HCL \cdot H_2O$ (768.83) Ber. C 65.6 H 7.2 N 5.5
Gef. C 65.8 H 7.1 N 5.4

Beispiel 40

1H-3,4-Dihydro-1-methyl-3-ethyl-3-[2-[4-[4-[2-(3,4,5-trimethoxyphenyl)-ethyl] -piperazinyl] -butoxy] -phenyl] - chinolin-2-on. Dihydrochlorid

Farblose Kristalle vom Schmp. 235 - 237° C

$^1$H-NMR (freie Base, $CDCl_3$): δ = 7.3-6.8 (m, 8H); 6.42 (s,2H) 4.0-3.8 (m,2H); 3.85+3,82 (2s,9H); 3.8+2.7 (AB-System,J= 15HZ, 2H); 3.42 (s,3H); 2.8-2.3 (m, 14H); 3.04 (2q, 2H); 1.8-1.6 (m,4H); 0.94 (t,3H)ppm.

$C_{37}H_{49}N_3O_5$ x 2HCL (688.74) Ber. C 64.5 H 7.4 N 6.1
Gef. C 64.4 H 7.4 N 6.1

Beispiel 41

1H-3,4-Dihydro-1-methyl-3-n-hexyl-3-[2-[4-[4-[2-(3,4,5-trimethoxyphenyl)-ethyl]-piperazinyl]- butoxy]-phenyl]-chinolin-2-on. Dihydrochlorid

Farblose Kristalle vom Schmp. 235 - 237° C

$^1$H-NMR (freie Base) $CDCl_3$): $\delta$ = 7.2-6.6 (m,8H); 6.4 (s,2H); 4.3-3.6 (m,3H); 3.82 (s,9H); 3.40 (s,3H); 2.9 - 2.2 (m,15H); 2.2-1,5 (m,6H); 1.4-1.0 (m,8H); 0.83 (t,3H) ppm

$C_{41}H_{57}N_3O_5$ x 2HCL (744,83) Ber. C 66.1 H 7.9 N 5.6
Gef. C 66.5 H 8.0 N 5.5

Beispiel 42

1H-3,4-Dihydro-1,3-dimethyl-3-[2-[4-[4-[2-(3,4,5-trimethoxyphenyl)-ethyl]-piperazinyl]-butoxy]-phenyl]-chinolin-2-on.Dihydrochlorid

Farblose Kristalle vom Schmp. 252 - 254° C

$^1$H-NMR (freie Base, $CDCl_3$): $\delta$ = 7.3-6.6 (m,8H9; 6.4 (s,2H); 4.1-3.6 (m,3H); 3.82 (s,9H); 3.40 (s,3H);2.7-2.1 (m,15H); 2.0-1,5 (m,4H); 1,50 (s,3H) ppm.

$C_{36}H_{47}N_3O_5$ x 2HCL (674.72) Ber. C 64.1 H 7.3 N 6.2
C 64.3 H 7.5 N 6.0

Beispiel 43

1H-3,4-Dihydro-1,3-dimethyl-3-[2-[4-[N-methyl-N-(2-(3,4-dimethoxyphenyl)-ethyl)-amino]-butoxy]-phenyl]-chinolin-2-on.Hydrochlorid

Farblose Kristalle vom Schmp. 107° C (Zersetzung)

1H-NMR (freie Base, $CDCl_3$): δ = 7.3-6.6 (m,11H); 4.2-3.6 (m,3H); 3.76 (s,6H); 3.30 (s,3H); 2.6-2.2 (m,6H); 2.13 (s,3H); 1.8-1.5 (m,4H); 1.44 (s,3H); ppm.

$C_{32}H_{40}N_2O_4$ x HCl x $2H_2O$ (589.18) Ber. C 65.2 H 7.7 N 4.6
C 65.2 H 7.5 N 4.5.

Die Verbindungen der folgenden Beispiele werden analog dem in Beispiel 37 beschriebenen Verfahren hergestellt, jedoch wird die freie Base jeweils in Aceton gelöst und mit equivalenten Mengen Oxalsäure versetzt. Nach Einengen wird mit Essigester/Ether kristallisiert.

Beispiel 44

1H-3,4-Dihydro-3-ethyl-1-methyl-3-[2-[4-[N-methyl-N-[2-(3,4-dimethoxyphenyl)-ethyl]-amino]-butoxy]-phenyl]-chinolin-2-on. Oxalat

Farblose Kristalle vom Schmp. 120° C

$^1$H-NMR ($CDCl_3$) freie Base): δ = 7.2-6.6 (m,11H); 4.1-3,5 (m,3H); 3,86 (s,6H); 3.40 (s,3H); 3.0-2,5 (m,7H); 2,30 (s,3H); 2,3-1.6 (m,6H); 0.93 (t,3H) ppm

$C_{33}H_{42}N_2O_4$ x $C_2H_2O_4$ (620,75) Ber. C 67.7 H 7.1 N 4.5
Gef. C 67.5 H 7.2 N 4.4

Beispiel 45

1H-3,4-Dihydro-3-n-hexyl-1-methyl-3-[2-[4-[N-methyl-N-[2-(3,4-dimethoxyphenyl)-ethyl]-amino]-butoxy]-phenyl]-chinolin-2-on.Oxalat

Farbloses amorphes Pulver

$^1$H-NMR (CDCl$_3$, freie Base): δ = 7.2-6.6 (m,11H); 4.0-3.6 (m,3H); 3.82 (s,6H); 3.40 (s,3H); 2.8-2.4 (m,7H); 2.27 (s,3H); 2.1-1.5 (m,6H); 1.5-1.0 (m,6H); 0.83 (t,3H) ppm.

$C_{37}H_{50}N_2O_4$ x $C_2H_2O_4$ x $H_2O$ (694.88) Ber C 67.4 H 7.8 N 4.0
Gef C 67.4 H 7.7 N 3.9

## Beispiel 46

1H-3.4-Dihydro-3-benzyl-1-methyl-3-[2-[4-[N-methyl-N-[2-(3,4-dimethoxyphenyl)-ethyl]-amino]-butoxy]-phenyl]-chinolin-2-on. Oxalat

Farblose Kristalle vom Schmp. 155° C

$^1$H-NMR (CDCl$_3$) freie Base): δ = 7.1-6.6 (m,16H); 4.1-3.7 (m,3H); 3.82 (s,6H); 3.53 + 3.38 (AB-System,2H); 3.32 (s,3H); 3.0-2.5 (m,7H); 2.43 (s,3H); 2.1-1.6 (m,4H) ppm.

$C_{38}H_{44}N_2O_4$ x $C_2H_2O_4$ (682.82) Ber. C 70.4 H 6.7 N 4.1
Gef. C 70.1 H 6.8 N 4.1

## Beispiel 47

1H-3.4-Dihydro-3-isopropyl-1-methyl-3-[2-[4-[N-methyl-N-[2-(3,4-dimethoxyphenyl)-ethyl]-amino]-butoxy]-phenyl]-chinolin-2-on-Oxalat

Farbloses amorphes Pulver

$^1$H-NMR (CDCl$_3$) freie Base): δ = 7.1-6.6 (m,11H); 4.1-3.6 (m,3H); 3.8 (s,6H); 3.4 (s,3H); 3.0-2.3 (m,8H); 2.4 (s,2H); 2.1-1.6 (m,4H); 1.1 + 0.9 (2d,6H) ppm.

Beispiel 48

1H-3.4-Dihydro-3-benzyl-1-methyl-3-[2-[4-[4-[4,4-bis-(4-fluorphenyl)-butyl]-piperazinyl]-butoxy]-phenyl]-chinolin-2-on. Dihydrochlorid

Hergestellt analog der in Beispiel 37 angegebenen Vorschrift aus equivalenten Mengen 1H-3,4-Dihydro-3-benzyl-1-methyl-3-[2-(4-brombutoxy)-phenyl]-chinolin-2-on und 1-[4,4-Bis-(4-fluorphenyl)-butyl]-piperazin.

Farblose Kristalle vom Schmp. 155° C (Zers.)

$^1$H-NMR (freie Base, $CDCl_3$): $\delta$ = 7.3-6.5 (m,21H); 4.2-3.9 (m,3H); 3.82 + 2.77 (AB-System, J=14H,2H); 3.63 + 3.35 (AB-System, J=13H,2H); 2.6-2.2 (m,14H); 2.2-1.2 (m,8H) ppm.

| | | | | | |
|---|---|---|---|---|---|
| $C_{47}H_{51}F_2N_3O_2$ x 2HCL x $H_2O$ (818.88) | Ber. | C 68.9 | H 6.8 | N 5.1 |
| | Gef. | C 69.2 | H 6.9 | N 5.2 |

Die Verbindungen der nachfolgenden Beispiele werden aus den entsprechenden Ausgangsmaterialien nach dem in Beispiel 48 angegebenen Verfahren hergestellt, jedoch werden die freien Basen in Aceton-Lösung mit 2.1 Equivalenten Maleinsäure versetzt.

Beispiel 49

1H-3,4-Dihydro-1,3-dimethyl-3-[2-[4-[4-[4,4-bis-(4-fluorphenyl)-butyl]-piperazinyl]-butoxy]-phenyl]-chinolin-2-on-Bis-Maleinat

Farblose Kristalle vom Schmp. 169-171° C (Zers.)

$^1$H-NMR (freie Pase, $CDCl_3$): $\delta$=7.3-6.6 (m,16H); 4.1-3.9 (m,3H); 3.82 + 2.57 (AB-System, J=10Hz,2H); 3.40 (s,3H); 2.5-2.0 (m,14H); 2.0-1.3 (m,8H); 1.50 (s,3H) ppm.

| | | | | | |
|---|---|---|---|---|---|
| $C_{41}H_{47}F_2N_3O_2 \times 2C_4H_4O_4$ (884.00) | Ber. | C 66.6 | H 6.3 | N 4.8 |
| | Gef. | C 66.5 | H 6.3 | N 4.8 |

Beispiel 50

1H-3.4-Dihydro-3-ethyl-1-methyl-3-[2-[4-[4-[4,4-bis-(4-fluorphenyl)-butyl]-piperazinyl]-butoxy]-phenyl]-chinolin-2-on-Bis-Maleinat

Farblose Kristalle vom Schmp. 169 - 171° C

$^1$H-NMR (freie Base,$CDCl_3$): $\delta$ = 7.3 - 6.6 (m,16H); 4.2-3.6 (m,4H); 3.38 (s,3H); 2.85-2.1 (m,15H); 2.05 (q,2H); 2.0-1.4 (m,8H); 0.93 (t,3H) ppm

| | | | | | |
|---|---|---|---|---|---|
| $C_{42}H_{49}F_2N_3O_2 \times 2C_4H_4O_4$ (898.02) | Ber. | C 66.9 | H 6.4 | N 4.7 |
| | Gef. | C 66.7 | H 6.7 | N 4.3 |

Beispiel 51

1H-3,4-Dihydro-3-n-hexyl-1-methyl-3-[2-[4-[4-[4,4-bis-(4-fluorphenyl)-butyl]-piperazinyl]-phenyl]-chinolin-2-on-Bis-Maleinat

Farblose Kristalle vom Schmp. 176 - 178° C

$^1$H-NMR (freie Base, $CDCl_3$): $\delta$ = 7.4-6.6 (m,16H); 4.2-3.6 (m,4H); 3.39 (s,3H); 2.85-2.2 (m,14H);2.1-1.4(m,10H);1.5-1.15 (m,8H); 0.85 (t,3H) ppm

$C_{46}H_{57}F_2N_3O_2 \times 2C_4H_4O_4$ (954.13) Ber. C 68.0 H 6.8 N 4.4
Gef. C 67.9 H 6.9 N 4.2

Nach den in den vorstehenden Beispielen angegebenen Vorschriften werden die in der nachstehenden Tabelle aufgeführten Verbindungen aus den entsprechenden Ausgangsmaterialien hergestellt.

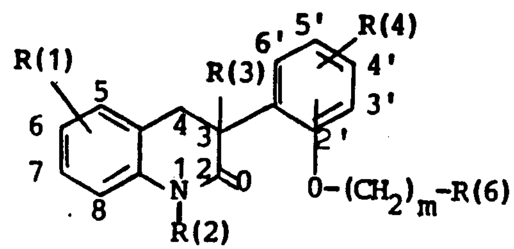

| Beisp. Nr. | R(1) | R(2) | R(3) | R(4) | m | R(6) | Seitenkette in Position | NMR (δ, ppm) |
|---|---|---|---|---|---|---|---|---|
| 52 | H | $CH_3$ | $CH(CH_3)_2$ | H | 4 | 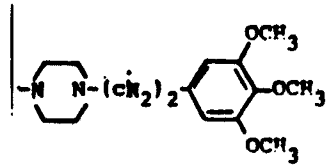 | 3' | 7.3-6.6(m,8H); 6.41(s,2H); 4.1-3.9(m,2H); 3.8+3.75 (2s,9H); 3.7-3.0 (m,2H); 3.4(s,3H); 2.9-2.4(m,15H); 2.0-1.5(m,4H); 1.0(d,6H) |
| 53 | H | $CH_3$ | $CH(CH_3)_2$ | H | 3 | " | 3' | 7.3-6.6(m,8H); 6.4(s,2H); 4.1-3.9(m,2H); 3.85+3.82 (2s,9H); 3.7-3.0(m,2H); 3.4(s,3H); 2.9-2.4(m,15H); 2.0-1.6(m,2H); 1.0(d,6H) |
| 54 | H | $CH_3$ | $CH(CH_3)_2$ | H | 3 | 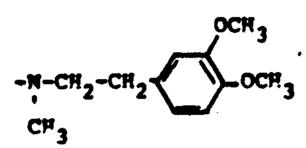 | 3' | 7.1-6.6(m,11H); 4.1-3.8 (m,3H); 3.8(s,6H); 3.4 (s,3H); 3.0-2.3(m,9H); 2.4 (s,3H); 2.1-1.7(m,2H); 1.0 (d,6H) |

| Beisp. Nr. | R(1) | R(2) | R(3) | R(4) | m | R(6) | Seitenkette in Position | NMR (δ, ppm) |
|---|---|---|---|---|---|---|---|---|
| 55 | H | $CH_3$ | $CH(CH_3)_2$ | H | 3 | 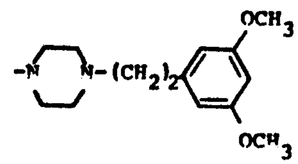 | 3' | 7.3-6.5(m,11H);4.1-3.9 (m,2H);3.7(s,6H);3.7-3.0 (m,2H);3.4(s,3H)2.9-2.4 (m,15H);2.0-1.6(m,2H);1.0 (d,6H) |
| 56 | H | $CH_3$ | $CH_2C_6H_5$ | H | 3 | wie Bsp. 52 | 3' | 7.2-6.5(m,13H);6.4(s,2H); 3.7(s,9H);4.2-3.2(m,5H); 3.0-2.2(m,15H);2.0-1.7 (m,2H) |
| 57 | H | $CH_3$ | $n-C_6H_{13}$ | H | 3 | " | 3' | 7.2-6.6(m,8H);6.4(s,2H) 4.2-3.6(m,3H);3.8(s,9H); 3.4(s,3H);2.9-2.2(m,15H); 2.2-1.5(m,4H);1.4-1.0 (m,8H);0,85(t,3H) |
| 58 | H | H | $CH(CH_3)_2$ | H | 4 | " | 2' | 7.2-6.5(m,8H);6.4(s,2H); 4.1-3.9(m,2H);3.85(s,9H); 3.7-3.0(m,2H);2.9-2.4 (m,15H);2.0-1.5(m,4H); 1.0(d,6H) |

| Beisp. Nr. | R(1) | R(2) | R(3) | R(4) | m | R(6) | Seitenkette in Position | NMR (δ, ppm) |
|---|---|---|---|---|---|---|---|---|
| 59 | H | $CH_3$ | $CH(CH_3)_2$ | H | 4 | 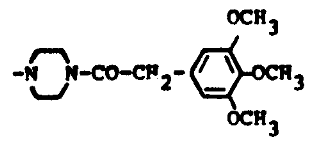 | 2' | 7.2-6.5(m,8H);6.5(s,2H); 4.1-3.9(m,2H);3.9(s,9H); 3.8(s,2H);3.7-3.0(m,2H); 3.4(s,3H);2.9-2.4(m,11H); 2.0-1.5(m,4H);1.0(d,6H) |
| 60 | H | $CH_3$ | $n-C_6H_{13}$ | H | 4 | " | 2' | 7.2-6.6(m,8H);6.5(s,2H); 4.1-3.6(m,3H);3.9(s,9H); 3.8(s,2H);3.4(s,3H);2.9-2.2(m,11H);2.2-1.5(m,6H); 1.4-1.0(m,8H);0.85(t,3H) |
| 61 | H | $CH_3$ | 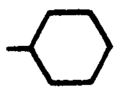 | H | 4 | wie Bsp. 52 | 2' | 7.2-6.6(m,8H);6.5(s,2H); 4.1-3.9(m,2H);3.85(s,9H); 3.7-3.0(m,2H);3.4(s,3H); 2.9-2.3(m,15H);2.2-1.3 (m,16H) |

| Beisp. Nr. | R(1) | R(2) | R(3) | R(4) | m | R(6) | Seitenkette in Position | NMR (δ, ppm) |
|---|---|---|---|---|---|---|---|---|
| 62 | H | $CH_3$ | $CH_2$- 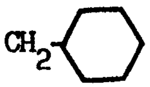 | H | 4 | wie Bsp.52 | 2' | 7.2-6.6(m,8H);6.5(s,2H); 4.1-3.9(m,2H);3.85(s,9H); 3.7-3.0(m,2H);3.4(s,3H); 2.9-2.3(m,15H);2.2-1.3 (m,16H) |
| 63 | H | $CH_3$ | $(CH_3)_2CH$ | H | 2 | " | 4' | 7.2-6.5(m,8H);6.5(s,2H); 4.1-3.9(m,2H);3.8(s,9H); 3.7-3.0(m,2H);3.4(s,3H); 2.9-2.4(m,15H);1.0(d,6H) |
| 64 | H | $CH_3$ | $(CH_3)_2CH$ | H | 3 | " | 4' | 7.2-6.5(m,8H);6.5(s,2H); 4.2-3.9(m,2H);3.85(s,9H); 3.75-3.05(m,2H);3.38(s,3H); 2.9-2.4(m,15H);2.0-1.6 (m,2H);1.0(d,6H) |
| 65 | H | $CH_3$ | $(CH_3)_2CH$ | H | 4 | " | 4' | 7.2-6.5(m,8H);6.4(s,2H); 4.2-3.8(m,2H);3.85(s,9H); 3.7-3.0(m,2H);3.4(s,3H); 2.9-2.4(m,15H);2.0-1.5 (m,4H);1.0(d,6H) |

-36-

| Beisp. Nr. | R(1) | R(2) | R(3) | R(4) | m | R(6) | Seitenkette in Position | NMR (δ, ppm) |
|---|---|---|---|---|---|---|---|---|
| 66 | H | $CH_3$ | $n-C_6H_{13}$ | H | 3 | wie Bsp. 52 | 4' | 7.2-6.5(m,8H);6.4(s,2H); 4.3-3.9(m,2H);3.85(s,9H); 3.7-3.0(m,2H);3.4(s,3H); 2.9-2.2(m,15H);2.2-1.5 (m,4H);1.4-1.0(m,8H);0.85 (t,3H) |
| 67 | H | $CH_3$ | $CH_3$ | H | 3 | " | 3' | 7.3-6.6(m,8H);6.4(s,2H); 4.1-3.6(m,3H);3.82(s,9H); 3.4(s,3H);2.7-2.1(m,15H); 2.0-1.5(m,2H);1.5(s,3H) |
| 68 | 6-Cl | $CH_3$ | $(CH_3)_2CH$ | H | 4 | " | 2' | 7.3-6.6(m,7H);6.4(s,2H); 4.1-3.9(m,2H);3.85(s,9H); 3.7-3.0(m,2H);3.4(s,3H); 2.9-2.4(m,15H);2.0-1.5(m,4H); 1.0(d,6H) |
| 69 | $6-CH_3$ | $CH_3$ | $(CH_3)_2CH$ | H | 4 | " | 2' | 7.3-6.6(m,7H);6.4(s,2H); 4.1-3.9(m,2H);3.85(s,9H); 3.7-3.1(m,2H);3.4(s,3H); 2.9-2.4(m,15H);2.3(s,3H); 2.0-1.5(m,4H)1.0(d,6H) |

| Beisp. Nr. | R(1) | R(2) | R(3) | R(4) | m | R(6) | Seitenkette in Position | NMR (δ, ppm) |
|---|---|---|---|---|---|---|---|---|
| 70 | 6-$OCH_3$ | $CH_3$ | $(CH_3)_2CH$ | H | 4 | wie Bsp. 52 | 2' | 7.3-6.5(m,7H);6.4(s,2H); 4.1-3.9(m,2H);3.9-3.75(3s 12H);3.7-3.1(m,2H);3,4(s, 3H);2.9-2.4(m,15H);2.0-1.5 (m,4H);1.0(d,6H) |
| 71 | 6,7-$(OCH_3)_2$ | $CH_3$ | $(CH_3)_2CH$ | H | 4 | " | 2' | 7.1-6.5(m,6H);6.4(s,2H); 4.1-3.9(m,2H);3.9-3.7(3s, 15H);3.7-3.0(m,2H);3.4(s, 3H);2.9-2.4(m,15H);2.0-1.5 (m,4H);1.0(d,6H) |
| 72 | 6,7-$OCH_2O$ | $CH_3$ | $(CH_3)_2CH$ | H | 4 | " | 2' | 7.1-6.5(m,6H);6.4(s,2H); 5.8(s,2H);4.1-3.9(m,2H); 3.85(s,9H);3.7-3.0(m,2H); 3.4(s,3H);2.9-2.4(m,15H); 2.0-1.5(m,4H);1.0(d,6H) |
| 73 | H | $CH_3$ | $(CH_3)_2CH$ | H | 4 | 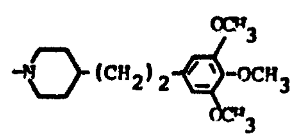 | 2' | 7.2-6.5(m,8H);6.4(s,2H); 4.1-3.9(m,2H);3.7-3.0(m, 2H);3.8(s,9H);3.4(s,3H); 2.9-2.4(m,10H); 2.0-1.5 (m,10H);1.0(d,6H) |

| Beisp. Nr. | R(1) | R(2) | R(3) | R(4) | m | R(6) | Seitenkette in Position | NMR (δ, ppm) |
|---|---|---|---|---|---|---|---|---|
| 74 | H | $CH_3$ | $(CH_3)_2CH$ | 5'-Cl | 4 | wie Bsp. 52 | 2' | 7.2-6.5(m,7H);6.4(s,2H); 4.1-3.9(m,2H);3.7-3.0(m, 2H);3.85(s,9H);3.4(s,3H); 2.9-2.4(m,15H);2.0-1.5(m, 4H);1.0(d,6H) |
| 75 | 6-Cl | $CH_3$ | $n-C_6H_{13}$ | H | 4 | " | 2' | 7.2-6.5(m,7H);6.4(s,2H); 4.1-3.9(m,2H);3.85(s,9H); 3.7-3.0(m,3H);3.4(s,3H); 2.9-2.4(m,15H);2.0-1.5 (m,6H);1.4-1.0(m,8H);0.85 (t,3H) |
| 76 | 6-Cl | $CH_3$ | $C_6H_5CH_2$ | H | 4 | " | 2' | 7.2-6.5(m,7H);6.4(s,2H); 4.2-3.6(m,3H);3.8(s,9H); 3.8-3.2(m,2H);3.4(s,3H); 3.0-2.2(m,15H);2.0-1.6(m,4H) |
| 77 | H | $CH_3$ | $(CH_3)_2CH$ | H | 5 | " | 2' | 7.2-6.5(m,8H);6.4(s,2H); 4.1-3.9(m,2H);3.8(s,9H); 3.7-3.0(m,2H);3.4(s,3H); 2.9-2.4(m,15H);2.0-1.5 m,8H);1.0(d,6H) |

| Beisp. Nr. | R(1) | R(2) | R(3) | R(4) | m | R(6) | Seitenkette in Position | NMR (δ, ppm) |
|---|---|---|---|---|---|---|---|---|
| 78 | 6-Cl | $CH_3$ | $(CH_3)_2CH$ | H | 4 | 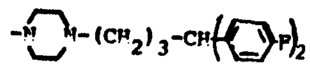 | 2' | 7.3-6.6(m,15H);4.1-3.9(m,3H);3.8-2.8(m,2H);3.4(s,3H);2.5-2.0(m,15H);2.0-1.3(m,8H);1.0(d,6H) |
| 79 | 6-$CH_3$ | $CH_3$ | $(CH_3)_2CH$ | H | 4 | " | 2' | 7.3-6.6(m,15H);4.1-3.9(m,3H);3.8-2.9(m,2H);3.4(s,3H);2.5-2.0(m,15H);2.3(s,3H);2.0-1.3(m,8H);1.0(d,6H) |
| 80 | 6-$OCH_3$ | $CH_3$ | $(CH_3)_2CH$ | H | 4 | " | 2' | 7.3-6.6(m,15H);4.1-3.9(m,3H);3.8-2.9(m,2H);3.8(s,3H);3.4(s,3H);2.5-2.0(m,15H);2.0-1.3(m,8H);1.0(d,6H) |
| 81 | H | $CH_3$ | $(CH_3)_2CH$ | H | 3 | 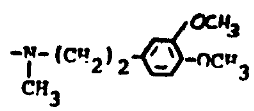 | 4' | 7.1-6.6(m,11H);4.1-3.8(m,3H);3.8(s,6H);3.4(s,3H);3.0-2.3(m,9H);2.4(s,3H);2.2-1.7(m,2H);1.0(d,6H) |

Beispiel 82
1H-3,4-Dihydro-3-isopropyl-1-methyl-3-[2-[2-hydroxy-3-[4-[2-(3,4,5-trimethoxyphenyl)-ethyl]-piperazinyl]-propoxy]-phenyl]-chinolin-2-on-Dihydrochlorid

a) 1-H-3,4-Dihydro-3-isopropyl-1-methyl-3-[2-(2,3-epoxy-propoxy)-phenyl]-chinolin-2-on

Hergestellt aus der Verbindung aus Beispiel 17 sowie Epichlorhydrin und Kaliumcarbonat analog der in Beispiel 25 angegebenen Vorschrift.
Farbloses Öl

$^1$H-NMR ($CDCl_3$): $\delta$ = 7.3-6.6 (m,8H); 5.0-4.0 (m,2H); 3.8-3.0 (m, 3H); 3.4 (s,3H); 1.0 (d,6H)

b) 1H-3,4-Dihydro-3-isopropyl-1-methyl-3-[2-[2-hydroxy-3-[4-[2-(3,4,5-trimethoxyphenyl)-ethyl]-piperazinyl]-propoxy]-phenyl]-chinolin-2-on-Dihydrochlorid

Jeweils 5 mmol des Epoxids aus a) und 2-(3,4,5-Trimethoxyphenyl)ethyl-piperazin werden in Toluol auf 80°C erwärmt. Nach Abkühlen wird eingeengt und an Kieselgel mit Methylenchlorid/Methanol (10:1) als Laufmittel chromatographiert.
Farbloses Öl

$^1$H-NMR ($CDCl_3$): $\delta$ = 7.3-6.6 (m,8H); 6.4 (s,2H); 4.4-4.1(m,2H); 4.0-3.0(m,3H); 3.85 (s,9H); 3,4 (s,3H); 3.0-2,4 (m,15H); 1.0(d,6H)

Die freie Base wird mit ethanolischer HCL in das Hydrochlorid überführt; farblose Kristalle.

Analog zu den in Beispiel 82 angegebenen Verfahren erhält man unter Verwendung der geeigneten Ausgangsmaterialien die folgenden Verbindungen:

Beispiel 83

1H-3,4-Dihydro-3-n-hexyl-1-methyl-3-[2-[2-hydroxy-3-[4-[2-(3,4,5-trimethoxyphenyl)-ethyl]-piperazinyl]-propoxy]-phenyl]-chinolin-2-on-Dihydrochlorid

Farblose Kristalle

$^1$H-NMR (freie Base, $CDCl_3$): $\delta$ = 7.2-6.6 (m,8H) 6.4 (s,2H); 4.0-3.0 (m,3H); 3.85 (s,9H); 3.4 (s,3H); 3.1-2.2 (m,14H); 2.2-1.5 (m, 2H); 1.4-1.0 (m,8H); 0.83 (t,3H)

Beispiel 84

1H-3,4-Dihydro-3-benzyl-1-methyl-3-[2-[2-hydroxy-3-[4-[2-(3,4,5-trimethoxyphenyl)-ethyl]piperazinyl]-propoxy]-phenyl]-chinolin-2-on-Dihydrochlorid

Farblose Kristalle

$^1$H-NMR (freie Base, $CDCl_3$): $\delta$ = 7.2-6.6 (m,13H); 6.4 (s, 2H); 4.4-4.1 (m,2H); 4.0-2.8 (m,5H); 3.85 (s,9H); 3.4 (s, 3H); 3.1-2.2 (m,15H);

Beispiel 85

1H-3,4-Dihydro-3-isopropyl-1-methyl-3-[2-[2-hydroxy-3-[N-methyl-N-[2-(3,4-dimethoxyphenyl)-ethyl]-amino]propoxy]-phenyl]-chinolin-2-on-Hydrochlorid

Farblose Kristalle

$^1$H-NMR (freie Base, $CDCl_3$): $\delta$ = 7.3-6.6 (m,11H); 4.4-3.6 (m,3H); 3.8 (s,6H); 3.3 (s,3H); 3.7-3.3 (m,1H); 2.6-2.2 (m,7H); 2.2 (s,3H); 1.0 (d,6H)

PATENTANSPRÜCHE:

1. Verbindung der Formel I

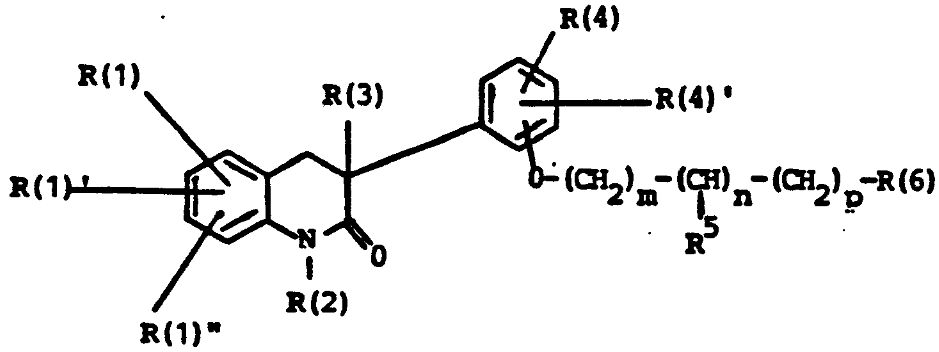

(I)

in welcher

R(1), R(1)' und R(1)'' gleich oder verschieden und voneinander unabhängig sind und Wasserstoff, $(C_1-C_4)$-Alkyl, $(C_1-C_3)$-Alkoxy, F, Cl, $CF_3$, Nitro, Hydroxy, Acetamido oder Amino, oder R(1) und R(1)' gemeinsam $(C_1-C_2)$-alkylendioxy,

R(2) Wasserstoff, $(C_1-C_{10}$-Alkyl, geradkettig oder verzweigt, $(C_3-C_{10})$-Alkenyl, geradkettig oder verzweigt,

R(3) Wasserstoff, $(C_1-C_{10})$-Alkyl, geradkettig oder verzweigt, $(C_3-C_{10})$-Alkenyl, geradkettig oder verzweigt, $(C_4-C_8)$-Cycloalkyl, $(C_4-C_8)$-Cycloalkyl-$(C_1-C_4)$-alkyl, Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)' gleich oder verschieden und voneinander unabhängig Wasserstoff, $(C_1-C_4)$-Alkyl, $(C_1-C_3)$-Alkoxy, F, Cl, $CF_3$, Nitro, Hydroxy, Acetamido oder Amino,

R(5) Wasserstoff, Hydroxy oder $(C_1-C_3)$-Alkyl,

m 1, 2, 3 oder 4,

n 0 oder 1,

p 0, 1, 2, 3 oder 4

bedeuten,

und

R(6) eine der folgenden Gruppen bedeutet,

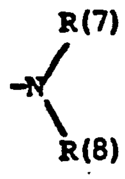 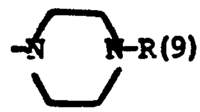 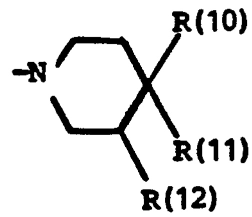 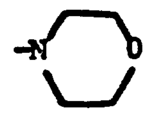

worin

R(7) und R(8) gleich oder verschieden und voneinander unabhängig Wasserstoff, $(C_1-C_{10})$-Alkyl, $(C_4-C_8)$-Cycloalkyl, $(C_4-C_8)$-Cycloalkyl-$(C_1-C_4)$-alkyl, Phenyl-$(C_1-C_6)$-alkyl, wobei der Phenylrest unsubstituiert oder durch einen, zwei oder drei Reste aus der Gruppe $(C_1-C_4)$-Alkylendioxy, F, Cl, $CF_3$ oder Hydroxy substituiert ist, Pyridyl-$(C_1-C_4)$-alkyl

R(9) Wasserstoff, $(C_1-C_{10})$-Alkyl, geradkettig oder verzweigt, Phenyl, Phenyl-$(C_1-C_4)$-alkyl oder Diphenyl-$(C_1-C_5)$-alkyl, Phenyl-$(C_1-C_4)$-alkanoyl, Benzoyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl, Pyrimidinyl, $(C_1-C_5)$-Alkanoyl,

R(10) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl oder Hydroxy substituiert ist,

R(11) Wasserstoff, Hydroxy, $(C_1-C_4)$-Alkoxy oder zusammen mit R(12) eine Bindung, und

R(12) Wasserstoff oder zusammen mit R(11) eine Bindung bedeuten;

sowie die Salze der Verbindungen der Formel I mit pharmazeutisch akzeptablen Säuren.

2. Verbindung I nach Anspruch 1, dadurch gekennzeichnet, daß

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido; oder

R(1)" Wasserstoff

R(2) Wasserstoff, $(C_1-C_6)$-Alkyl, geradkettig oder verzweigt,

R(3) Wasserstoff, $(C_1-C_6)$-Alkyl, geradkettig oder verzweigt, Allyl, Cyclopentyl, Cyclohexyl, Benzyl, Phenylethyl, jeweils unsubstituiert oder durch $(C_1-C_3)$-Alkyl, $(C_1-C_3)$-Alkoxy, F, Cl, $CF_3$, $(C_1-C_2)$-Alkylendioxy oder Nitro substituiert,

R(4) Wasserstoff, Methyl, Methoxy, Ethoxy, Chlor, Nitro, Hydroxy, Acetamido oder Amino,

R(4)' Wasserstoff,

R(5) Wasserstoff oder Hydroxy,

m 1, 2 oder 3,

n 0 oder 1,

p 1, 2, 3 oder 4 bedeuten und

R(6) eine der folgenden Gruppen

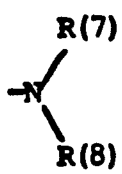

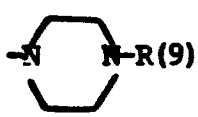

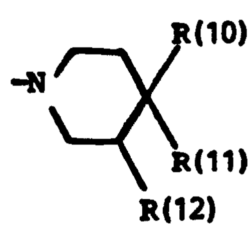

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-$(C_1-C_4)$-alkyl, wobei der Phenylrest unsubstituiert oder durch einen, zwei oder drei Reste aus der Gruppe $(C_1-C_4)$-Alkyl, $(C_1-C_4)$-Alkoxy, $(C_1-C_2)$-Alkylen-

dioxy, F, Cl, $CF_3$ oder Hydroxy substituiert ist, Pyridyl-$(C_1-C_4)$-alkyl,

R(9) Wasserstoff, $(C_1-C_{10})$-Alkyl, geradkettig oder verzweigt, Phenyl, Phenyl-$(C_1-C_4)$-alkyl oder Diphenyl-$(C_1-C_5)$-alkyl, Phenyl-$(C_1-C_4)$-alkanoyl, Benzoyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl, Pyrimidinyl, $(C_1-C_5)$-Alkanoyl,

R(10) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl oder Hydroxy substituiert ist,

R(11) Wasserstoff und Hydroxy, Methoxy oder zusammen mit R(12) eine Bindung,

R(12) Wasserstoff oder zusammen mit R(11) eine Bindung bedeuten.

3. Verbindung I nach mindestens einem der Ansprüche 1 bis 2, dadurch gekennzeichnet, daß

R(1) Wasserstoff, Methyl, Methoxy, Fluor oder Chlor,

R(1)' und R(1)'' Wasserstoff,

R(2) Wasserstoff, Methyl, Ethyl,

R(3) Wasserstoff, Methyl, Ethyl, Propyl, Isopropyl, Butyl, sec. Butyl, Isobutyl, Allyl, Cyclopentyl, Cyclohexyl, Benzyl, Phenylethyl, wobei der Phenylring jeweils unsubstituiert oder durch Methyl, Methoxy, $CF_3$, Fluor Chlor, Methylendioxy, Nitro substituiert ist,

R(4) Wasserstoff, Methyl, Methoxy, Chlor, Nitro oder Hydroxy,

R(4)' Wasserstoff,

R(5) Wasserstoff, Hydroxy oder $(C_1-C_3)$-Alkyl

m 0 oder 1,

n 0 oder 1,

p 1, 2, 3 oder 4

bedeuten, und
R(6) eine der folgenden Gruppen bedeutet

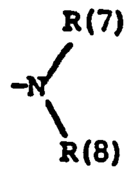 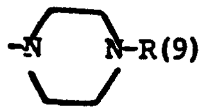 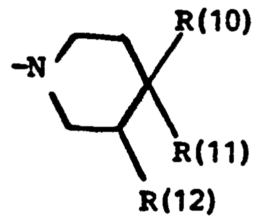

worin

R(7) Wasserstoff oder Methyl,

R(8) Phenyl-($C_1$-$C_4$)-alkyl, wobei der Phenylrest unsubstituiert oder durch einen, zwei oder drei Reste aus der Gruppe Methyl, Methoxy, Chlor, Methylendioxy oder Hydroxy substituiert ist,

R(9) Wasserstoff, ($C_1$-$C_{10}$)-Alkyl, geradkettig oder verzweigt, Phenyl, Phenyl-($C_1$-$C_4$)-alkyl oder Diphenyl-($C_1$-$C_5$)-alkyl, Phenyl-($C_1$-$C_4$)-alkanoyl, Benzoyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist, Pyridyl, Pyrimidinyl, ($C_1$-$C_5$)-Alkanoyl,

R(10) Phenyl oder Phenyl-($C_1$-$C_2$)-alkyl, wobei der Phenylrest jeweils unsubstituiert oder durch einen, zwei oder drei Reste aus der Gruppe Methyl, Methoxy, Chlor, Methylendioxy oder Hydroxy substituiert ist,

R(11) Wasserstoff, Hydroxy, Methoxy oder zusammen mit R(12) eine Bindung, und

R(12) Wasserstoff oder zusammen mit R(11) eine Bindung bedeuten.

4. Verbindung I nach mindestens einem der Ansprüche 1 bis 3, dadurch gekennzeichnet, daß

R(1) Wasserstoff, Methyl, Methoxy, Fluor oder Chlor,
R(1)' und R(1)" Wasserstoff,
R(2) Wasserstoff, Methyl,
R(3) Methyl, Ethyl, Propyl, Isopropyl, Butyl, sec. Butyl, Isobutyl, Cyclopentyl, Cyclohexyl, Benzyl, unsubstituiert oder durch Methoxy, Methyl, Fluor, Chlor, oder Nitro substituiert,
R(4) Wasserstoff, Methoxy, Methyl oder Chlor,
R(5) Wasserstoff oder Hydroxy,
m 0 oder 1
n 0 oder 1
p 1, 2, 3 oder 4
R(6) eine der folgenden Gruppen

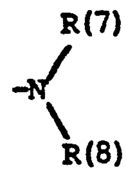

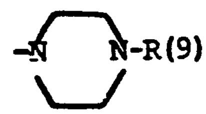

R(7) Methyl,
R(8) Phenyl-($C_1$-$C_4$)-alkyl, wobei der Phenylrest unsubstituiert oder durch einen, zwei oder drei Reste aus der Gruppe Methyl, Methoxy, Chlor, Methylendioxy oder Hydroxy substituiert ist,
R(9) Phenyl-($C_1$-$C_4$)-alkyl, wobei der Phenylrest unsubstituiert oder durch einen, zwei oder drei Reste aus der Gruppe ($C_1$-$C_2$)-Alkoxy, ($C_1$-$C_2$)-Alkylendioxy oder Hydroxy substituiert ist, Diphenyl-($C_1$-$C_4$)-alkyl, wobei die Phenylreste unsubstituiert oder durch Chlor, Fluor oder Methoxy substituiert sind.

5. Verfahren zur Herstellung der Verbindung I nach Anspruch 1, dadurch gekennzeichnet, daß man

a) eine Verbindung der Formel II,

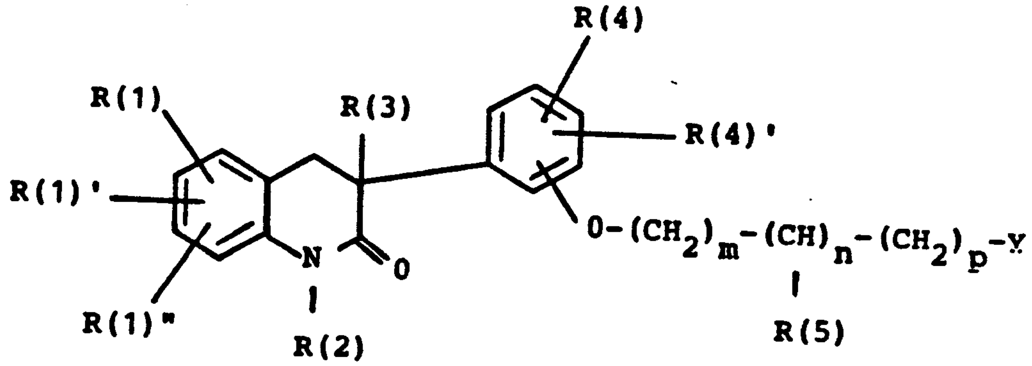

(II)

in welcher R(1), R(1)', R(1)", R(2), R(3), R(4), R(4)', R(5), m, n und p die gleiche Bedeutung wie in Formel I haben, und in welcher Y eine Abgangsgruppe, die nucleophil verdrängt werden kann, mit einer der Verbindungen der Formeln IIIa, IIIb, IIIc oder IIId,

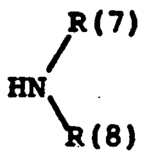

(IIIa)

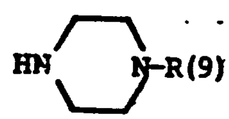

(IIIb)

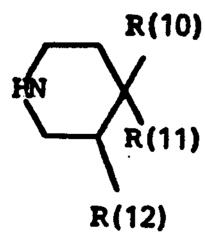

(IIIc)

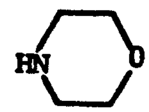

(IIId)

in welchen R(7), R(8), R(9), R(10), R(11) und R(12) die gleiche Bedeutung wie in Formel I haben, unter Bedingungen einer nucleophilen Substitution umsetzt, oder daß man

b) eine Verbindung der Formel IV,

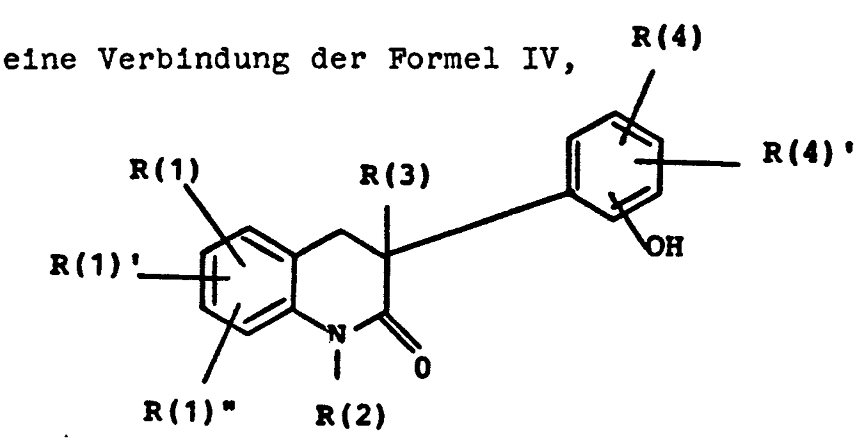

(IV)

in welcher R(1), R(1)', R(1)", R(2), R(3), R(4), R(4)' die gleiche Bedeutung wie in Formel I haben, mit einer Verbindung der Formel V,

$$Z\text{-}(CH_2)_m\text{-}\underset{\substack{|\\ R(5)}}{(CH)_n}\text{-}(CH_2)_p\text{-}R(6) \qquad (V)$$

in welcher Z gleich wie Y in Formel II definiert ist und in welcher R(5), R(6), m, n und p die gleiche Bedeutung wie in Formel I haben, entweder in einem polaren aprotischen Lösungsmittel in Gegenwart einer starken Base bei einer Temperatur zwischen -40 und +60°C, vorzugsweise zwischen -10 und +30°C, oder in einem protischen oder aprotischen polaren organischen Lösungsmittel in Gegenwart einer schwachen bis mittelstarken Base bei einer Temperatur zwischen 0 und 160°C, vorzugsweise zwischen 20 und 120°C, umsetzt.

6. Verbindung der Formel II

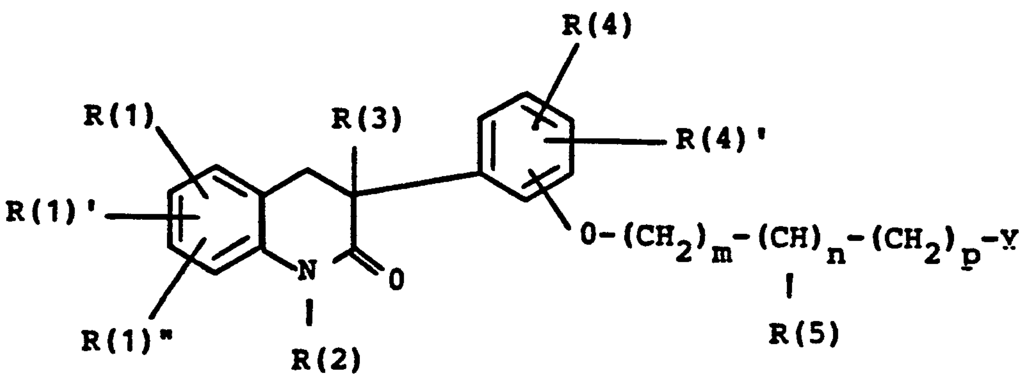

(II)

in welcher R(1), R(1)', R(1)", R(2), R(3), R(4), R(4)', R(5), m, n und p die in Anspruch I genannten Bedeutungen haben und Y eine Abgangsgruppe, die nucleophil verdrängt werden kann, bedeutet.

7. Verfahren zum Herstellen einer Verbindung II nach Anspruch 6, dadurch gekennzeichnet, daß man einen 2-Nitrobenzaldehyd der Formel VI

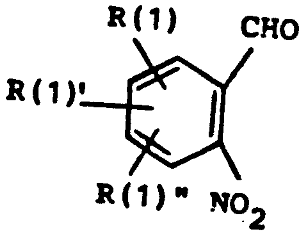

(VI)

in welcher R(1), R(1)' und R(1)" die oben genannten Bedeutungen haben, mit einer Verbindung der Formel VII

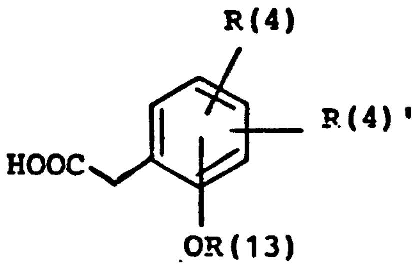

(VII)

in welcher R(4) und R(4)' die obengenannte Bedeutung haben und R(13) einen unter milden Bedingungen abspaltbaren Rest bedeuten,
in Essigsäureanhydrid zur Verbindung der Formel VIII

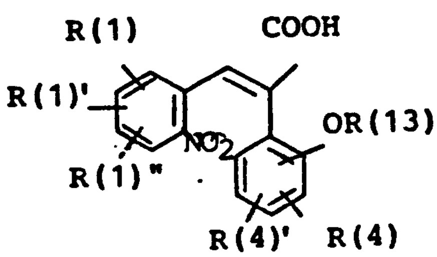

VIII

umsetzt,
und diese zur Verbindung der Formel IX

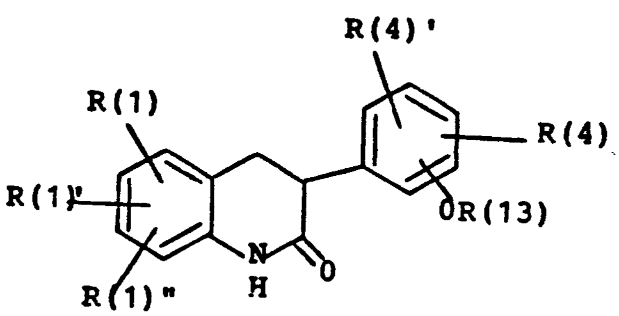

IX

hydriert,
die Verbindung der Formel IX am Stickstoffatom zur Verbindung der Formel X

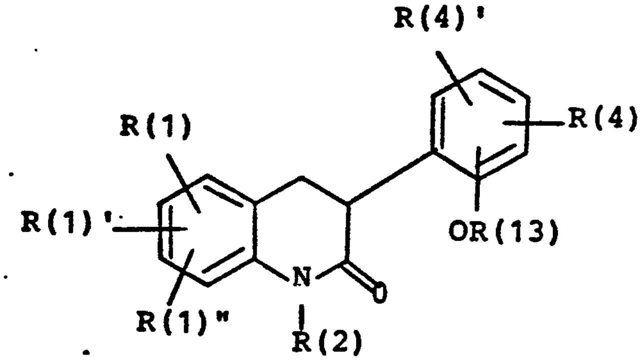

X

alkyliert,
gegebenenfalls zur Verbindung XI

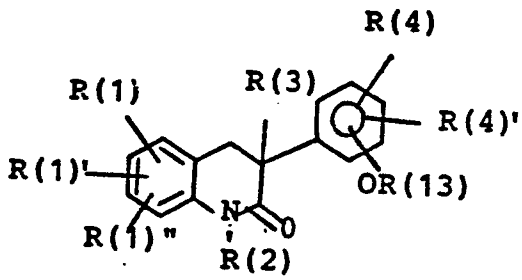

XI

alkyliert,
danach die Schutzgruppe R(13) aus Verbindung X oder XI abspaltet und so Verbindung IV

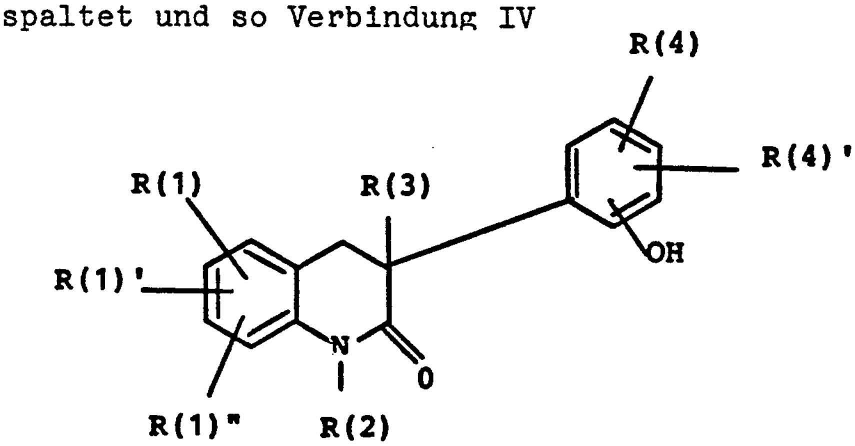

(IV)

erhält
und daß man sodann mit einer Verbindung der Formel XII

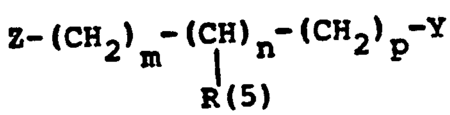

XII

in welcher R(5), m, n und Y die obengenannte Bedeutung haben zur Verbindung der Formel II umsetzt.

8. Mittel zum Behandeln von Erkrankungen des Herz-Kreislaufsystems, dadurch gekennzeichnet, daß es mindestens eine Verbindung der Formel I nach Anspruch 1 zusammen mit Trägerstoffen enthält.

9. Verwendung einer Verbindung der Formel I nach Anspruch 1 zum Behandeln von Erkrankungen des Herz-Kreislaufsystems.

10. Verwendung einer Verbindung I nach Anspruch 1 zur Herstellung eines Arzneimittels zur Behandlung von Erkrankungen des Herz-Kreislaufsystems.

Patentansprüche Österreich:

1. Verfahren zur Herstellung einer Verbindung der Formel I

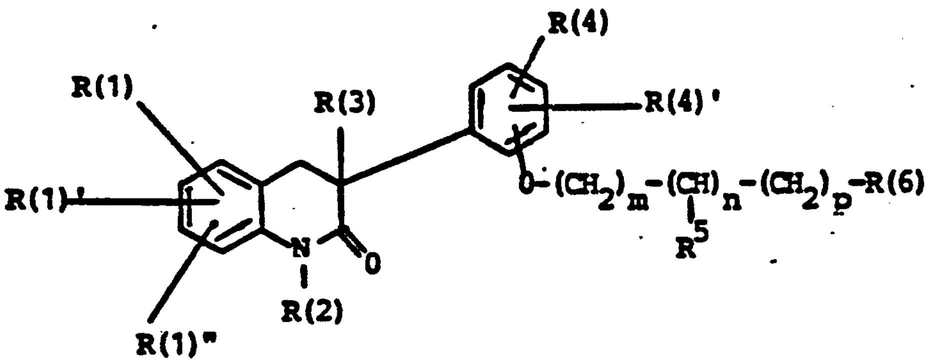

(I),

in welcher

R(1), R(1)' und R(1)'' gleich oder verschieden und voneinander unabhängig sind und Wasserstoff, $(C_1-C_4)$-Alkyl, $(C_1-C_3)$-Alkoxy, F, Cl, $CF_3$, Nitro, Hydroxy, Acetamido oder Amino, oder R(1) und R(1)' gemeinsam $(C_1-C_2)$-alkylendioxy,

R(2) Wasserstoff, $(C_1-C_{10}$-Alkyl, geradkettig oder verzweigt, $(C_3-C_{10})$-Alkenyl, geradkettig oder verzweigt,

R(3) Wasserstoff, $(C_1-C_{10})$-Alkyl, geradkettig oder verzweigt, $(C_3-C_{10})$-Alkenyl, geradkettig oder verzweigt, $(C_4-C_8)$-Cycloalkyl, $(C_4-C_8)$-Cycloalkyl-$(C_1-C_4)$-alkyl, Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)' gleich oder verschieden und voneinander unabhängig Wasserstoff, $(C_1-C_4)$-Alkyl, $(C_1-C_3)$-Alkoxy, F, Cl, $CF_3$, Nitro, Hydroxy, Acetamido oder Amino,

R(5) Wasserstoff, Hydroxy oder $(C_1-C_3)$-Alkyl,

m 1, 2, 3 oder 4,

n 0 oder 1,

p 0, 1, 2, 3 oder 4

bedeuten,

und

R(6) eine der folgenden Gruppen bedeutet,

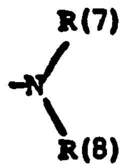 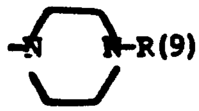 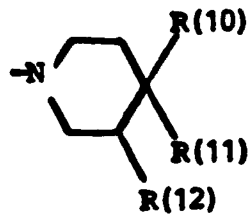 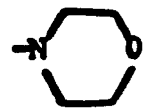

worin

R(7) und R(8) gleich oder verschieden und voneinander unabhängig Wasserstoff, $(C_1-C_{10})$-Alkyl, $(C_4-C_8)$-Cycloalkyl, $(C_4-C_8)$-Cycloalkyl-$(C_1-C_4)$-alkyl, Phenyl-$(C_1-C_6)$-alkyl, wobei der Phenylrest unsubstituiert oder durch einen, zwei oder drei Reste aus der Gruppe $(C_1-C_4)$-Alkylendioxy, F, Cl, $CF_3$ oder Hydroxy substituiert ist, Pyridyl-$(C_1-C_4)$-alkyl

R(9) Wasserstoff, $(C_1-C_{10})$-Alkyl, geradkettig oder verzweigt, Phenyl, Phenyl-$(C_1-C_4)$-alkyl oder Diphenyl-$(C_1-C_5)$-alkyl, Phenyl-$(C_1-C_4)$-alkanoyl, Benzoyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl, Pyrimidinyl, $(C_1-C_5)$-Alkanoyl,

R(10) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl oder Hydroxy substituiert ist,

R(11) Wasserstoff, Hydroxy, $(C_1-C_4)$-Alkoxy oder zusammen mit R(12) eine Bindung, und

R(12) Wasserstoff oder zusammen mit R(11) eine Bindung bedeuten;

sowie die Salze der Verbindungen der Formel I mit pharmazeutisch akzeptablen Säuren,

dadurch gekennzeichnet, daß man

a) eine Verbindung der Formel II

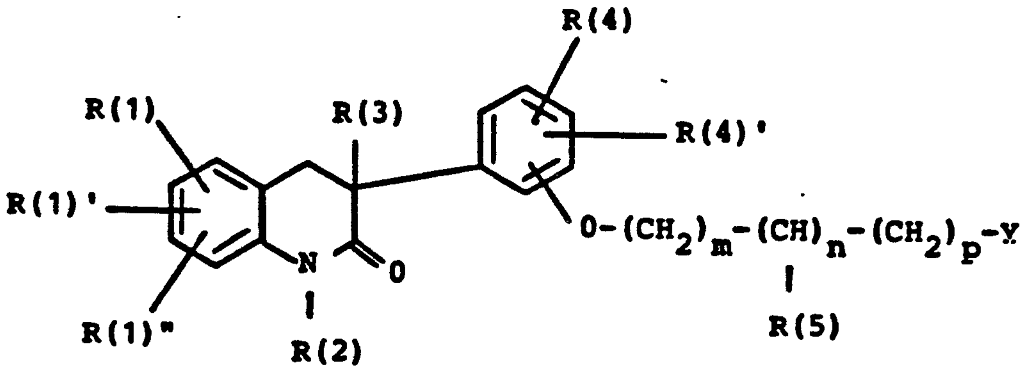
(II)

in welcher R(1), R(1)', R(1)", R(2), R(3), R(4), R(4)', R(5), m, n und p die gleiche Bedeutung wie in Formel I haben, und in welcher Y eine Abgangsgruppe, die nucleophil verdrängt werden kann,
mit einer der Verbindungen der Formeln IIIa, IIIb, IIIc oder IIId,

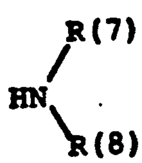
(IIIa)

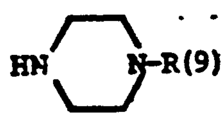
(IIIb)

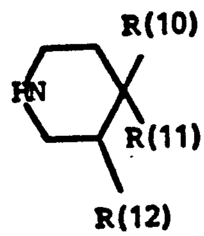
(IIIc)

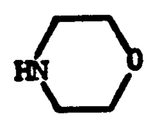
(IIId)

in welchen R(7), R(8), R(9), R(10), R(11) und R(12) die gleiche Bedeutung wie in Formel I haben, unter Bedingungen einer nucleophilen Substitution
umsetzt, oder daß man

b) eine Verbindung der Formel IV,

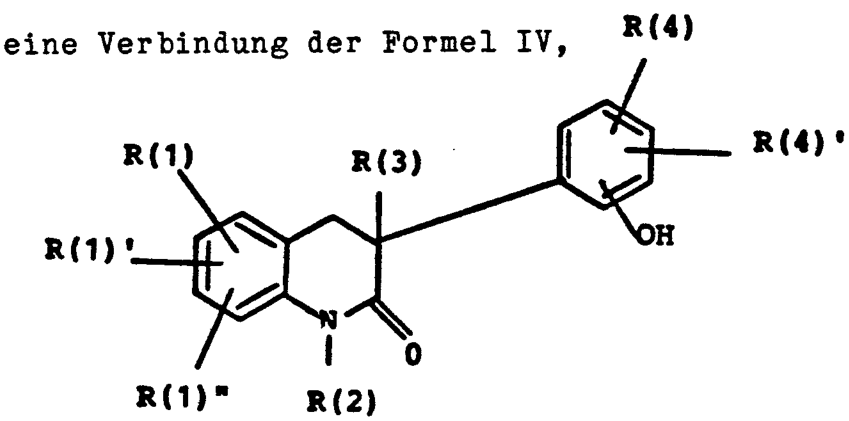
(IV)

in welcher R(1), R(1)', R(1)", R(2), R(3), R(4), R(4)' die gleiche Bedeutung wie in Formel I haben, mit einer Verbindung der Formel V,

$$Z-(CH_2)_m-\underset{R(5)}{(CH)_n}-(CH_2)_p-R(6) \qquad (V)$$

in welcher Z gleich wie Y in Formel II definiert ist und in welcher R(5), R(6), m, n und p die gleiche Bedeutung wie in Formel I haben, entweder in einem polaren aprotischen Lösungsmittel in Gegenwart einer starken Base bei einer Temperatur zwischen -40 und +60°C, vorzugsweise zwischen -10 und +30°C, oder in einem protischen oder aprotischen polaren organischen Lösungsmittel in Gegenwart einer schwachen bis mittelstarken Base bei einer Temperatur zwischen 0 und 160°C, vorzugsweise zwischen 20 und 120°C, umsetzt.

2. Verfahren zur Herstellung einer Verbindung I nach Anspruch 1, dadurch gekennzeichnet, daß

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido; oder
R(1)" Wasserstoff
R(2) Wasserstoff, $(C_1-C_6)$-Alkyl, geradkettig oder verzweigt,
R(3) Wasserstoff, $(C_1-C_6)$-Alkyl, geradkettig oder verzweigt, Allyl, Cyclopentyl, Cyclohexyl, Benzyl, Phenylethyl, jeweils unsubstituiert oder durch $(C_1-C_3)$-Alkyl, $(C_1-C_3)$-Alkoxy, F, Cl, $CF_3$, $(C_1-C_2)$-Alkylendioxy oder Nitro substituiert,
R(4) Wasserstoff, Methyl, Methoxy, Ethoxy, Chlor, Nitro, Hydroxy, Acetamido oder Amino,
R(4)' Wasserstoff,
R(5) Wasserstoff oder Hydroxy,

m 1, 2 oder 3,

n 0 oder 1,

p 1, 2, 3 oder 4 bedeuten und

R(6) eine der folgenden Gruppen

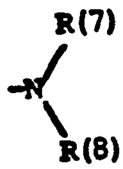 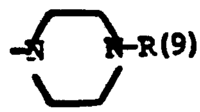 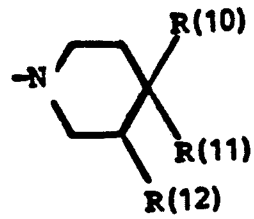

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-($C_1$-$C_4$)-alkyl, wobei der Phenylrest unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist, Pyridyl-($C_1$-$C_4$)-alkyl,

R(9) Wasserstoff, ($C_1$-$C_{10}$)-Alkyl, geradkettig oder verzweigt, Phenyl, Phenyl-($C_1$-$C_4$)-alkyl oder Diphenyl-($C_1$-$C_5$)-alkyl, Phenyl-($C_1$-$C_4$)-alkanoyl, Benzoyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist, Pyridyl, Pyrimidinyl, ($C_1$-$C_5$)-Alkanoyl,

R(10) Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl oder Hydroxy substituiert ist,

R(11) Wasserstoff und Hydroxy, Methoxy oder zusammen mit R(12) eine Bindung,

R(12) Wasserstoff oder zusammen mit R(11) eine Bindung bedeuten.

3. Verfahren zur Herstellung einer Verbindung I nach einem der Ansprüche 1 und 2, dadurch gekennzeichnet, daß

R(1) Wasserstoff, Methyl, Methoxy, Fluor oder Chlor,
R(1)' und R(1)'' Wasserstoff,
R(2) Wasserstoff, Methyl, Ethyl,
R(3) Wasserstoff, Methyl, Ethyl, Propyl, Isopropyl, Butyl, sec. Butyl, Isobutyl, Allyl, Cyclopentyl, Cyclohexyl, Benzyl, Phenylethyl, wobei der Phenylring jeweils unsubstituiert oder durch Methyl, Methoxy, $CF_3$, Fluor Chlor, Methylendioxy, Nitro substituiert ist,
R(4) Wasserstoff, Methyl, Methoxy, Chlor, Nitro oder Hydroxy,
R(4)' Wasserstoff,
R(5) Wasserstoff, Hydroxy oder $(C_1-C_3)$-Alkyl
m 0 oder 1,
n 0 oder 1,
p 1, 2, 3 oder 4
bedeuten, und
R(6) eine der folgenden Gruppen bedeutet

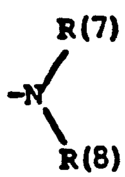
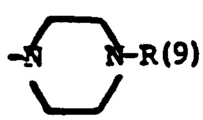
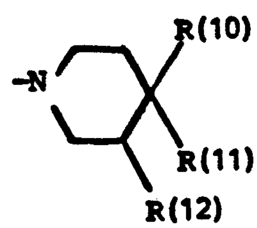

worin

R(7) Wasserstoff oder Methyl,
R(8) Phenyl-$(C_1-C_4)$-alkyl, wobei der Phenylrest unsubstituiert oder durch einen, zwei oder drei Reste aus der Gruppe Methyl, Methoxy, Chlor, Methylendioxy oder Hydroxy substituiert ist,

R(9) Wasserstoff, $(C_1-C_{10})$-Alkyl, geradkettig oder verzweigt, Phenyl, Phenyl-$(C_1-C_4)$-alkyl oder Diphenyl-$(C_1-C_5)$-alkyl, Phenyl-$(C_1-C_4)$-alkanoyl, Benzoyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Pyridyl, Pyrimidinyl, $(C_1-C_5)$-Alkanoyl,

R(10) Phenyl oder Phenyl-$(C_1-C_2)$-alkyl, wobei der Phenylrest jeweils unsubstituiert oder durch einen, zwei oder drei Reste aus der Gruppe Methyl, Methoxy, Chlor, Methylendioxy oder Hydroxy substituiert ist,

R(11) Wasserstoff, Hydroxy, Methoxy oder zusammen mit R(12) eine Bindung, und

R(12) Wasserstoff oder zusammen mit R(11) eine Bindung bedeuten.

4. Verfahren zur Herstellung einer Verbindung I nach einem der Ansprüche 1 bis 3, dadurch gekennzeichnet, daß

R(1) Wasserstoff, Methyl, Methoxy, Fluor oder Chlor,

R(1)' und R(1)" Wasserstoff,

R(2) Wasserstoff, Methyl,

R(3) Methyl, Ethyl, Propyl, Isopropyl, Butyl, sec. Butyl, Isobutyl, Cyclopentyl, Cyclohexyl, Benzyl, unsubstituiert oder durch Methoxy, Methyl, Fluor, Chlor, oder Nitro substituiert,

R(4) Wasserstoff, Methoxy, Methyl oder Chlor,

R(5) Wasserstoff oder Hydroxy,

m 0 oder 1

n 0 oder 1

p 1, 2, 3 oder 4

R(6) eine der folgenden Gruppen

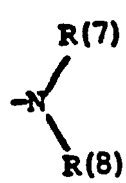

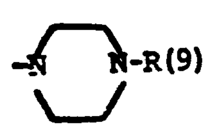

R(7) Methyl,

R(8) Phenyl-($C_1$-$C_4$)-alkyl, wobei der Phenylrest unsubstituiert oder durch einen, zwei oder drei Reste aus der Gruppe Methyl, Methoxy, Chlor, Methylendioxy oder Hydroxy substituiert ist,

R(9) Phenyl-($C_1$-$C_4$)-alkyl, wobei der Phenylrest unsubstituiert oder durch einen, zwei oder drei Reste aus der Gruppe ($C_1$-$C_2$)-Alkoxy, ($C_1$-$C_2$)-Alkylendioxy oder Hydroxy substituiert ist, Diphenyl-($C_1$-$C_4$)-alkyl, wobei die Phenylreste unsubstituiert oder durch Chlor, Fluor oder Methoxy substituiert sind.

5. Verfahren zum Herstellen einer Verbindung II

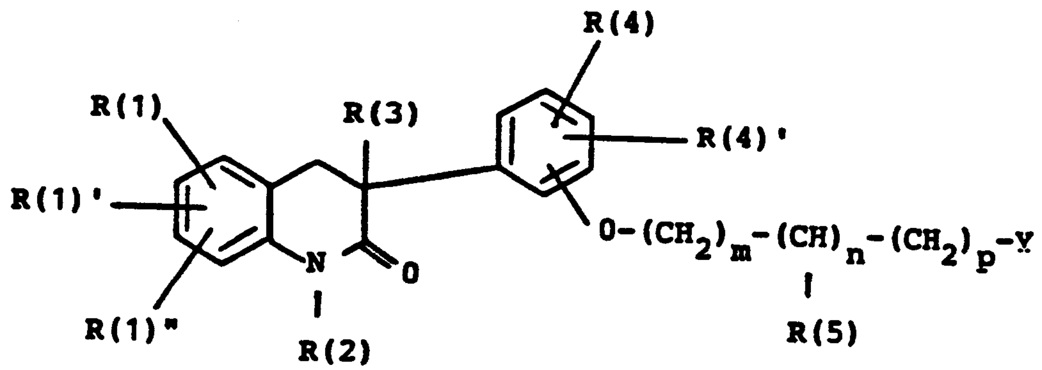 (II)

in welcher R(1), R(1)', R(1)", R(2), R(3), R(4), R(4)', R(5), m, n und p die in Anspruch 1 genannten Bedeutungen haben und Y eine Abgangsgruppe, die nucleophil verdrängt werden kann, bedeutet, dadurch gekennzeichnet, daß man einen 2-Nitrobenzaldehyd der Formel VI

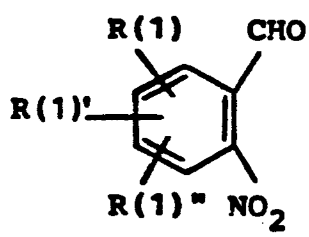 (VI)

in welcher R(1), R(1)' und R(1)" die oben genannten Bedeutungen haben, mit einer Verbindung der Formel VII

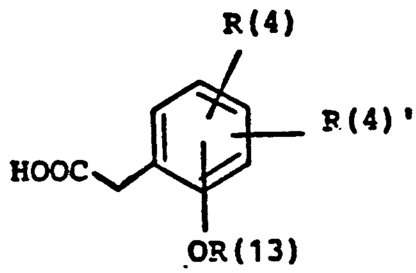

(VII)

in welcher R(4) und R(4)' die obengenannte Bedeutung haben und R(13) einen unter milden Bedingungen abspaltbaren Rest bedeuten,
in Essigsäureanhydrid zur Verbindung der Formel VIII

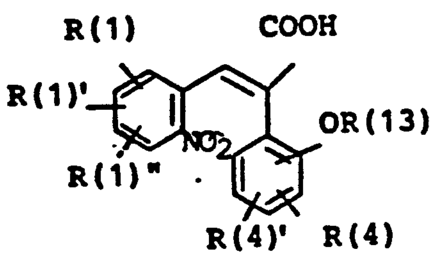

VIII

umsetzt,
und diese zur Verbindung der Formel IX

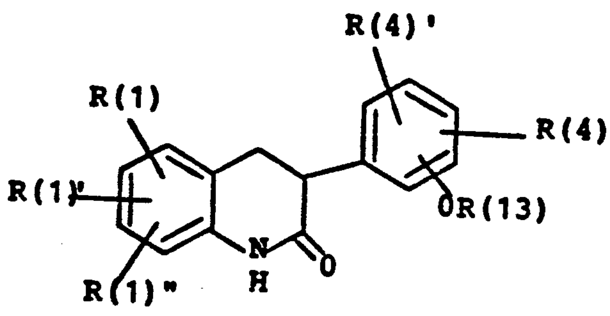

IX

hydriert,
die Verbindung der Formel IX am Stickstoffatom zur Verbindung der Formel X

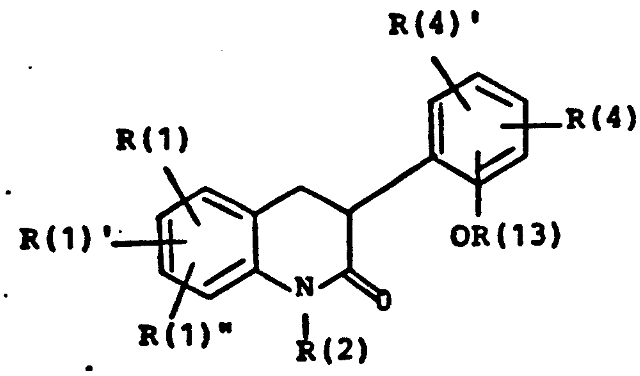

X

alkyliert,
gegebenenfalls zur Verbindung XI

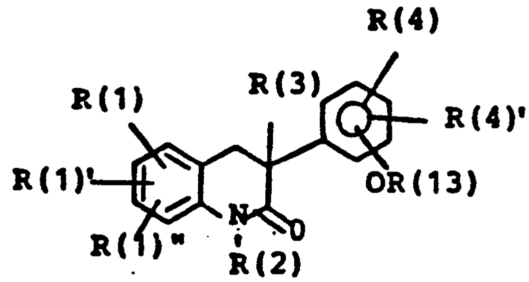

XI

alkyliert,
danach die Schutzgruppe R(13) aus Verbindung X oder XI abspaltet und so Verbindung IV

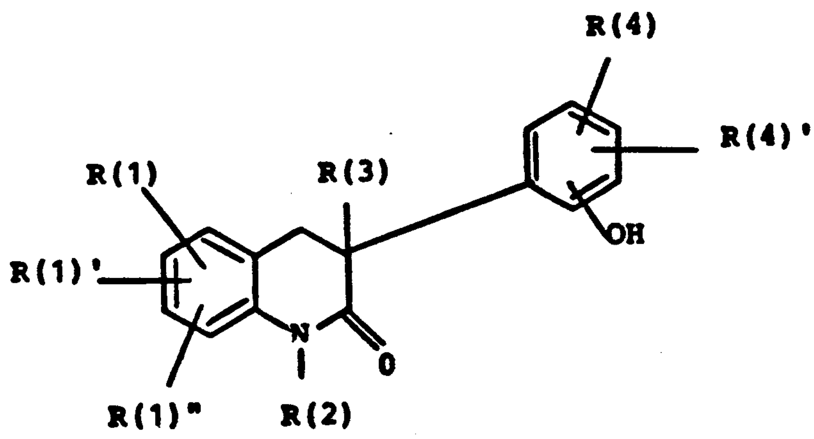

(IV)

erhält,
und daß man sodann mit einer Verbindung der Formel XII

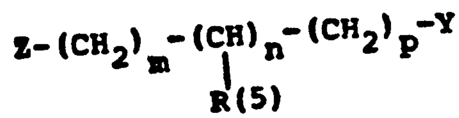

XII

in welcher R(5), m, n und Y die obengenannte Bedeutung haben zur Verbindung der Formel II umsetzt.

6. Verfahren zum Herstellen eines Mittels zum Behandeln von Erkrankungen des Herz-Kreislaufsystems, dadurch gekennzeichnet, daß man mindestens eine Verbindung der Formel I nach Anspruch 1 mit Trägerstoffen zusammenmischt.

0212216

Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 86109476.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB - A - 1 042 638 (CASSELLA)<br>* Ansprüche 1,3; Seite 2, Zeilen 19-23 *<br>-- | 1,8,10 | C 07 D 215/22<br>C 07 D 401/12<br>C 07 D 491/056<br>A 61 K 31/47 |
| D,A | GB - A - 1 305 278 (SQUIBB)<br>* Ansprüche 1,10; Seite 3, Zeilen 10-18 *<br>-- | 1,6,7 | |
| A | US - A - 4 071 520 (WEHRMEISTER)<br>---- | | |

RECHERCHIERTE SACHGEBIETE (Int. Cl.4)

C 07 D 215/00
C 07 D 401/00
C 07 D 491/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,10
Unvollständig recherchierte Patentansprüche: -
Nicht recherchierte Patentansprüche: 9
Grund für die Beschränkung der Recherche:

Verfahren zur therapeutischen Behandlung des menschlichen oder tierischen Körpers; Artikel 52(4) EPÜ

| Recherchenort | Abschlußdatum der Recherche | Prüfer |
|---|---|---|
| WIEN | 24-10-1986 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82